# EUROPEAN PATENT SPECIFICATION

(11) **EP 3 004 047 B1**
(45) Date of publication and mention of the grant of the patent: **22.02.2017**
(21) Application number: 14733718.2
(22) Date of filing: 05.06.2014
(51) Int. Cl.: C07C 201/08, C07C 205/56, C07D 471/04

(54) **COMPOUNDS OF '3-(5-SUBSTITUTED OXY-2,4-DINITRO-PHENYL)-2-OXO-PROPIONIC ACID ESTER', PROCESS AND APPLICATIONS THEREOF**
VERBINDUNGEN VON '3-(5-SUBSTITUIERTEN OXY-2,4-DINITRO-PHENYL)-2-OXO-PROPIONSÄUREESTER, VERFAHREN UND ANWENDUNGEN DAVON
COMPOSÉS DE L'"ESTER DE L'ACIDE 3-(OXY-2,4-DINITRO-PHÉNYLE 5-SUBSTITUÉ)-2-OXO-PROPIONIQUE", PROCÉDÉ POUR LEUR PRÉPARATION ET LEURS APPLICATIONS

(30) Priority: 06.06.2013 IN CH24662013
(43) Date of publication of application: 13.04.2016
(73) Proprietor: Anthem Biosciences Pvt Ltd, Bangalore 56099, Karnataka (IN)
(72) Inventor: GAVARA GOVINDA, Rajulu, Bangalore 560 099 Karnataka (IN); SAMBASIVAM, Ganesh, Bangalore 560 034 Karnataka (IN); PUTHIAPARAMPIL, Tom Thomas, Bangalore 560 078 Karnataka (IN); CHANDRAPPA KORAMANGALA, Ravindra, Bangalore 560 034 Karnataka (IN)
(74) Representative: V.O.
(86) International application number: PCT/IB2014/061980
(87) International publication number: WO 2014/195896

(56) References cited:
- WO-A1-96/21644
- ROBERT BUJOK ET AL: "Expedient Synthesis of 1-Hydroxy-4- and 1-Hydroxy-6-nitroindoles", SYNLETT, vol. 23, no. 9, 14 May 2012 (2012-05-14), pages 1315-1320, XP055135680, DE ISSN: 0936-5214, DOI: 10.1055/s-0031-1291044

## Description

### TECHNICAL FIELD

The present disclosure relates to compounds of Formula I and process of obtaining the same. Said compounds of Formula I have numerous applications, more particularly, in the synthesis of pyrroloquinoline quinone (PQQ), 4,5-dioxo-4,5-dihydro-1H-pyrrolo[2,3-f]quinoline-2,7,9-tricarboxylic acid 2-allyl ester, 5-ethoxy-5-hydroxy-4-oxo-4,5-dihydro-1H-pyrrolo[2,3-f]quinoline-2,7,9-tricarboxylic acid, 5-hydroxy-6-(1,1,4-trioxo-1lambda*6*-1,2,5-thiadiazolidin-2-yl)-1H-indole-2-carboxylic acid and their pharmaceutically acceptable salts.

Further, the synthesis of PQQ and salts thereof is carried out via. compounds of Formula I as intermediate as represented below: 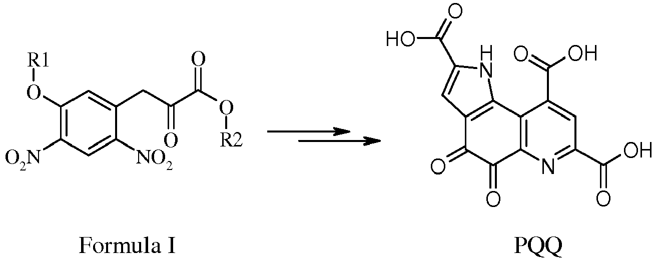 wherein, R1 and R2 is individually selected from a group comprising hydrogen, straight or branched chain C₁₋₈ alkyl, straight or branched chain C₁₋₈ alkenyl, straight or branched chain C₁₋₈ alkynyl, aralkyl, substituted aralkyl, heteroaralkyl, substituted heteroaralkyl and each of them can be optionally substituted.

Similarly, the synthesis of 4,5-dioxo-4,5-dihydro-1H-pyrrolo[2,3-f]quinoline-2,7,9-tricarboxylic acid 2-allyl ester, 5-ethoxy-5-hydroxy-4-oxo-4,5-dihydro-1H-pyrrolo[2,3-f]quinoline-2,7,9-tricarboxylic acid, 5-hydroxy-6-(1,1,4-trioxo-1lambda*6*-1,2,5-thiadiazolidin-2-yl)-1H-indole-2-carboxylic acid and salts thereof is carried out via. compounds of Formula I as intermediate.

The said synthesis of PQQ, 4,5-dioxo-4,5-dihydro-1H-pyrrolo[2,3-f]quinoline-2,7,9-tricarboxylic acid 2-allyl ester, 5-ethoxy-5-hydroxy-4-oxo-4,5-dihydro-1H-pyrrolo[2,3-f]quinoline-2,7,9-tricarboxylic acid, 5-hydroxy-6-(1,1,4-trioxo-1lambda*6*-1,2,5-thiadiazolidin-2-yl)-1H-indole-2-carboxylic acid via. Formula I compounds result in shorter process employing minimum number of steps and result in improved efficiency and higher yield among other advantages.

### BACKGROUND AND PRIOR ART

Pyrroloquinoline quinone (PQQ) is a natural product and is also known as methoxatin. Among many applications, primary use of PQQ is to protect mitochondria from oxidative stress, providing neuroprotection and cardioprotection. Common food sources of PQQ are parsley, green pepper, green tea, papaya, kiwi and milk. However, the available concentrations of PQQ in the food sources are only in picomolar (pM) to nanomolar (nM) levels. This necessitates the development of chemical processes which can produce large quantities of PQQ.

The first complete synthesis of PQQ was reported by Corey et al. [J. Am. Chem. Soc. 103 (1981), 5599 -5600]. As per the Corey publication, PQQ was synthesized in milligram (mg) scale and the number of steps involved in the synthesis of PQQ from commercially available starting material were ten.

Later, the synthesis of PQQ by a nine step-method was reported by Martin et al., Helv, Chem, Acta 76 (1993), 1667. However, the synthetic route described here is very similar to the aforementioned Corey's route and isolation of the final compound involves very complex and laborious workup procedure.

Kempf et al., (WO2006/102642A1) describes the synthesis of PQQ. This application mainly discloses the large scale synthesis of PQQ via. a combination of Corey's and Martin's route. The application further claims the purification of final compound with sulphuric acid. However, the synthesis method involves large number of steps and the isolation of advanced intermediates further involves tedious workup procedure. WO96/21644 discloses in preparation 8 on page 27 the synthesis of ethyl-*alpha*-oxo-3-(2-nitro-4,5-dichlorophenyl)propanoate from 3-nitro-4,5-dichlorotoluene and diethyloxalate.

Hence, it can be observed that there is an immense need for better and simpler synthetic routes for obtaining pyrroloquinoline quinone (PQQ) and related compounds. The present disclosure aims at overcoming the drawbacks of prior art and provide with Formula I compounds which can be employed in the synthesis of PQQ and related compounds. Thus, the present disclosure is able to provide for improved and cost effective synthetic routes having minimal steps for the production of said compounds.

### STATEMENT OF THE DISCLOSURE

Accordingly, the present disclosure relates to a compound of Formula I 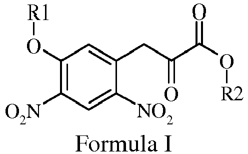 wherein, R1 and R2 are individually selected from a group comprising hydrogen, straight or branched chain C₁₋₈ alkyl, straight or branched chain C₁₋₈ alkenyl, straight or branched chain C₁₋₈ alkynyl, aralkyl, substituted aralkyl, alkaryl, substituted alkaryl, heteroaralkyl, substituted heteroaralkyl and wherein each of them may be optionally substituted, or tautomers or salts thereof; a process of obtaining a compound of Formula I 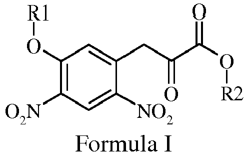 wherein, R1 and R2 are individually selected from a group comprising hydrogen, straight or branched chain C₁₋₈ alkyl, straight or branched chain C₁₋₈ alkenyl, straight or branched chain C₁₋₈ alkynyl, aralkyl, substituted aralkyl, alkaryl, substituted alkaryl, heteroaralkyl, substituted heteroaralkyl and wherein each of them may be optionally substituted,
or tautomers or salts thereof, said process comprising reacting a compound of Formula III with ester and base to obtain the compound of Formula I 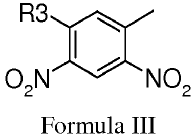 wherein R3 is selected from a group comprising fluoro, chloro, bromo, iodo, methanesulfonyloxy, toluenesulfonyloxy, trifluoromethanesulfonyloxy, alkyloxy, aryloxy, arolkyl oxy, heteroaryloxy and wherein each of them may be optionally substituted; and
a process for preparing a compound of Formula IV via Formula I, or tautomers or salts thereof, 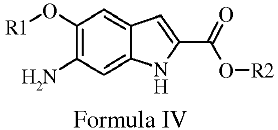 wherein, R1 and R2 are individually selected from a group comprising hydrogen, straight or branched chain C₁₋₈ alkyl, straight or branched chain C₁₋₈ alkenyl, straight or branched chain C₁₋₈ alkynyl, aralkyl, substituted aralkyl, alkaryl, substituted alkaryl, heteroaralkyl, substituted heteroaralkyl and wherein each of them may be optionally substituted, said process comprising steps of:
a. reacting a compound of Formula II with nitration mixture to obtain a compound of Formula III 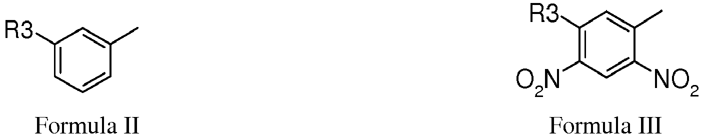 wherein, R3 is selected from a group comprising fluoro, chloro, bromo, iodo, methanesulfonyloxy, toluenesulfonyloxy, trifluoromethanesulfonyloxy, alkyloxy, aryloxy, arolkyl oxy, heteroaryloxy and wherein each of them may be optionally substituted;
b. reacting the compound of Formula III with ester and base to obtain a compound of Formula I, or tautomers or salts thereof 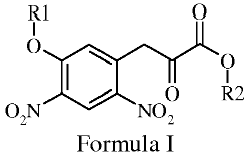 wherein, R1 and R2 are individually selected from a group comprising hydrogen, straight or branched chain C₁₋₈ alkyl, straight or branched chain C₁₋₈ alkenyl, straight or branched chain C₁₋₈ alkynyl, aralkyl, substituted aralkyl, alkaryl, substituted alkaryl, heteroaralkyl, substituted heteroaralkyl and wherein each of them may be optionally substituted; and
c. reacting the compound of Formula I with reducing agent to obtain a compound of Formula IV 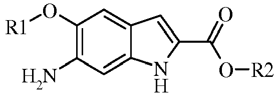 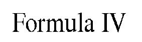 wherein, R1 and R2 are individually selected from a group comprising hydrogen, straight or branched chain C₁₋₈ alkyl, straight or branched chain C₁₋₈ alkenyl, straight or branched chain C₁₋₈ alkynyl, aralkyl, substituted aralkyl, alkaryl, substituted alkaryl, heteroaralkyl, substituted heteroaralkyl and wherein each of them may be optionally substituted.

### BRIEF DESCRIPTION OF ACCOMPANYING FIGURES

**Figure 1** depicts the instant method of synthesizing 4,5-dioxo-4,5-dihydro-1H-pyrrolo[2,3-f]quinoline-2,7,9-tricarboxylic acid (PQQ) and corresponding salts.
**Figure 2** depicts the truncated synthesis path for PQQ formation.
**Figure 3** depicts the instant method of synthesizing 5-hydroxy-6-(1,1,4-trioxo-1lambda*6*-1,2,5-thiadiazolidin-2-yl)-1H-indole-2-carboxylic acid and corresponding salts.
**Figure 4** depicts the instant method of synthesizing 4,5-dioxo-4,5-dihydro-1H-pyrrolo[2,3-f]quinoline-2,7,9-tricarboxylic acid 2-allyl ester and corresponding salts.
**Figure 5** depicts the instant method of synthesizing 5-ethoxy-5-hydroxy-4-oxo-4,5-dihydro-1H-pyrrolo[2,3-f]quinoline-2,7,9-tricarboxylic acid and corresponding salts.

### DETAILED DESCRIPTION OF THE DISCLOSURE

The present disclosure relates to compound of Formula I 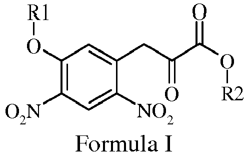 wherein, R1 and R2 are individually selected from a group comprising hydrogen, straight or branched chain C₁₋₈ alkyl, straight or branched chain C₁₋₈ alkenyl, straight or branched chain C₁₋₈ alkynyl, aralkyl, substituted aralkyl, alkaryl, substituted alkaryl, heteroaralkyl, substituted heteroaralkyl and wherein each of them may be optionally substituted,
or tautomers or salts thereof.

The present disclosure further relates to a process of obtaining a compound of Formula I 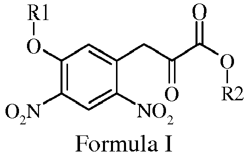 wherein, R1 and R2 are individually selected from a group comprising hydrogen, straight or branched chain C₁₋₈ alkyl, straight or branched chain C₁₋₈ alkenyl, straight or branched chain C₁₋₈ alkynyl, aralkyl, substituted aralkyl, alkaryl, substituted alkaryl, heteroaralkyl, substituted heteroaralkyl and wherein each of them may be optionally substituted,
or tautomers or salts thereof, said process comprising reacting a compound of Formula III with ester and base to obtain the compound of Formula I 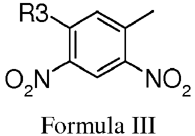 wherein R3 is selected from a group comprising fluoro, chloro, bromo, iodo, methanesulfonyloxy, toluenesulfonyloxy, trifluoromethanesulfonyloxy, alkyloxy, aryloxy, arolkyl oxy, heteroaryloxy and wherein each of them may be optionally substituted.

In an embodiment of the present disclosure, the ester as mentioned above is oxalic acid ester selected from a group comprising dimethyl oxalate, diallyl oxalate, diethyl oxalate, dibenzyl oxalate, diisopropyl oxalate and di-tert-butyl oxalate or any combination thereof; and the base is selected from a group comprising sodium methoxide, sodium ethoxide, sodium benzyloxide, potassium hydroxide, potassium *t*-butoxide, sodium hydride and 1,8-diazabicyclo[5.4.0]undec-7-ene or any combination thereof.

In an embodiment of the present disclosure, the above process is carried out at temperature ranging from about -20 °C to about 100 °C, preferably about 0 °C to about 40 °C and for a time period ranging from about 60 minutes to about 72 hours, preferably about 60 minutes to about 16 hours.

In another embodiment of the present disclosure, the above process further comprises isolation of the compound of Formula I; and wherein the isolation is carried out by acts selected from a group comprising quenching, filtration and extraction or any combination of acts in any order thereof.

The present disclosure further relates to a process for preparing a compound of Formula IV via Formula I, or tautomers or salts thereof, 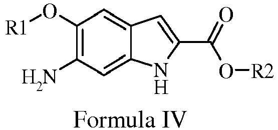 wherein, R1 and R2 are individually selected from a group comprising hydrogen, straight or branched chain C₁₋₈ alkyl, straight or branched chain C₁₋₈ alkenyl, straight or branched chain C₁₋₈ alkynyl, aralkyl, substituted aralkyl, alkaryl, substituted alkaryl, heteroaralkyl, substituted heteroaralkyl and wherein each of them may be optionally substituted, said process comprising steps of:
a. reacting a compound of Formula II with nitration mixture to obtain a compound of Formula III 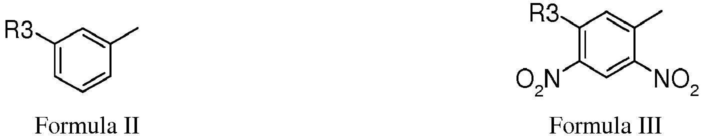 wherein, R3 is selected from a group comprising fluoro, chloro, bromo, iodo, methanesulfonyloxy, toluenesulfonyloxy, trifluoromethanesulfonyloxy, alkyloxy, aryloxy, arolkyl oxy, heteroaryloxy and wherein each of them may be optionally substituted;
b. reacting the compound of Formula III with ester and base to obtain a compound of Formula I, or tautomers or salts thereof 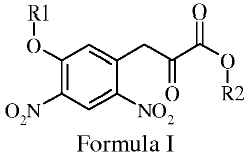 wherein, R1 and R2 are individually selected from a group comprising hydrogen, straight or branched chain C₁₋₈ alkyl, straight or branched chain C₁₋₈ alkenyl, straight or branched chain C₁₋₈ alkynyl, aralkyl, substituted aralkyl, alkaryl, substituted alkaryl, heteroaralkyl, substituted heteroaralkyl and wherein each of them may be optionally substituted; and
c. reacting the compound of Formula I with reducing agent to obtain a compound of Formula IV 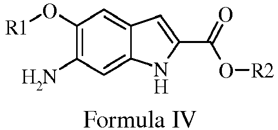 wherein, R1 and R2 are individually selected from a group comprising hydrogen, straight or branched chain C₁₋₈ alkyl, straight or branched chain C₁₋₈ alkenyl, straight or branched chain C₁₋₈ alkynyl, aralkyl, substituted aralkyl, alkaryl, substituted alkaryl, heteroaralkyl, substituted heteroaralkyl and wherein each of them may be optionally substituted.

In an embodiment of the present disclosure, wherein the nitration mixture as disclosed above is selected from a group comprising a mixture of nitric acid and sulphuric acid, calcium nitrate and acetic acid, and tert-butyl nitrite or any combination of mixtures thereof; the ester in step (b) is oxalic acid ester selected from a group comprising dimethyl oxalate, diallyl oxalate, diethyl oxalate, Di-*tert*-butyl oxalate, and diisopropyl oxalate or any combination thereof; the base in step (b) is selected from a group comprising sodium methoxide, sodium benzyloxide, sodium ethoxide, potassium hydroxide, potassium *t*-butoxide, sodium hydride and 1,8-diazabicyclo[5.4.0]undec-7-ene or any combination thereof; the reducing agent in step (c) is selected from a group comprising palladium, palladium on carbon, nickel, sodium dithionite, iron/hydrochloric acid, zinc/acetic acid and tin chloride/hydrochloric acid or any combination thereof.

In another embodiment of the present disclosure, the step c) of the above described process further comprises acts of:
i. converting the compound of Formula IV to a compound of Formula V in presence of coupling agent 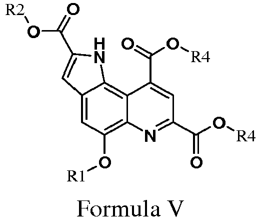 wherein, R1 and R2 are individually selected from a group comprising hydrogen, straight or branched chain C₁₋₈ alkyl, straight or branched chain C₁₋₈ alkenyl, straight or branched chain C₁₋₈ alkynyl, aralkyl, substituted aralkyl, alkaryl, substituted alkaryl, heteroaralkyl, substituted heteroaralkyl and wherein each of them may be optionally substituted; and R4 is selected from group comprising, but not limited to, hydrogen, straight or branched chain C₁₋₈ alkyl, straight or branched chain C₁₋₈ alkenyl, straight or branched chain C₁₋₈ alkynyl, aralkyl, substituted aralkyl, heteroaralkyl, substituted heteroaralkyl and wherein each of them may be optionally substituted;
ii. reacting the compound of Formula V with oxidizing agent to obtain a compound of Formula VI 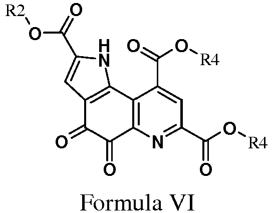 wherein R2 is selected from a group comprising hydrogen, straight or branched chain C₁₋₈ alkyl, straight or branched chain C₁₋₈ alkenyl, straight or branched chain C₁₋₈ alkynyl, aralkyl, substituted aralkyl, alkaryl, substituted alkaryl, heteroaralkyl, substituted heteroaralkyl and wherein each of them may be optionally substituted; and R4 is selected from group comprising hydrogen, straight or branched chain C₁₋₈ alkyl, straight or branched chain C₁₋₈ alkenyl, straight or branched chain C₁₋₈ alkynyl, aralkyl, substituted aralkyl, heteroaralkyl, substituted heteroaralkyl and wherein each of them may be optionally substituted; and
iii. hydrolyzing the compound of Formula VI to obtain 4,5-dioxo-4,5-dihydro-1H-pyrrolo[2,3-f]quinoline-2,7,9-tricarboxylic acid (PQQ) or 4,5-dioxo-4,5-dihydro-1H-pyrrolo[2,3-f]quinoline-2,7,9-tricarboxylic acid 2-allyl ester, or salts thereof.

In yet another embodiment of the present disclosure, the step c) of the above described process further comprises acts of:
i. converting the compound of Formula IV to a compound of Formula VII in presence of chlorosulfonyl isocyanate 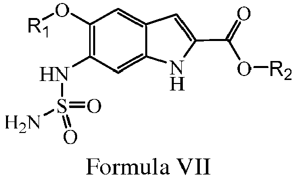 wherein, R1 and R2 are individually selected from a group comprising hydrogen, straight or branched chain C₁₋₈ alkyl, straight or branched chain C₁₋₈ alkenyl, straight or branched chain C₁₋₈ alkynyl, aralkyl, substituted aralkyl, alkaryl, substituted alkaryl, heteroaralkyl, substituted heteroaralkyl and wherein each of them may be optionally substituted; and R4 is selected from group comprising, but not limited to, hydrogen, straight or branched chain C₁₋₈ alkyl, straight or branched chain C₁₋₈ alkenyl, straight or branched chain C₁₋₈ alkynyl, aralkyl, substituted aralkyl, heteroaralkyl, substituted heteroaralkyl and wherein each of them may be optionally substituted; and
ii. reacting the compound of Formula VII with ethyl bromoacetate and palladium on carbon to obtain a compound of 5-hydroxy-6-(1,1,4-trioxo-llambda*6*-1,2,5-thiadiazolidin-2-yl)-1H-indole-2-carboxylic acid or salts thereof.

In still another embodiment of the present disclosure, the step c) of the above described process further comprises acts of:
i. converting the compound of Formula IV to a compound of Formula V in presence of coupling agent 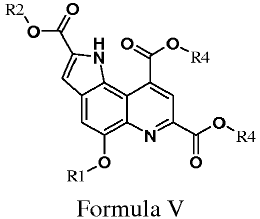 wherein, R1 and R2 are individually selected from a group comprising hydrogen, straight or branched chain C₁₋₈ alkyl, straight or branched chain C₁₋₈ alkenyl, straight or branched chain C₁₋₈ alkynyl, aralkyl, substituted aralkyl, alkaryl, substituted alkaryl, heteroaralkyl, substituted heteroaralkyl and wherein each of them may be optionally substituted; and R4 is selected from group comprising, but not limited to, hydrogen, straight or branched chain C₁₋₈ alkyl, straight or branched chain C₁₋₈ alkenyl, straight or branched chain C₁₋₈ alkynyl, aralkyl, substituted aralkyl, heteroaralkyl, substituted heteroaralkyl and wherein each of them may be optionally substituted;
ii. reacting the compound of Formula V with oxidizing agent to obtain a compound of Formula VI 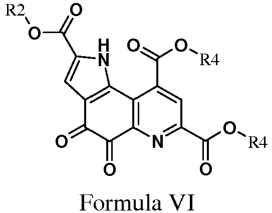 wherein R2 is selected from a group comprising hydrogen, straight or branched chain C₁₋₈ alkyl, straight or branched chain C₁₋₈ alkenyl, straight or branched chain C₁₋₈ alkynyl, aralkyl, substituted aralkyl, alkaryl, substituted alkaryl, heteroaralkyl, substituted heteroaralkyl and wherein each of them may be optionally substituted; and R4 is selected from group comprising hydrogen, straight or branched chain C₁₋₈ alkyl, straight or branched chain C₁₋₈ alkenyl, straight or branched chain C₁₋₈ alkynyl, aralkyl, substituted aralkyl, heteroaralkyl, substituted heteroaralkyl and wherein each of them may be optionally substituted;
iii. hydrolyzing the compound of Formula VI to obtain 4,5-dioxo-4,5-dihydro-1H-pyrrolo[2,3-f]quinoline-2,7,9-tricarboxylic acid (PQQ) or salts thereof; and
iv. refluxing the 4,5-dioxo-4,5-dihydro-1H-pyrrolo[2,3-f]quinoline-2,7,9-tricarboxylic acid (PQQ) in presence of ethanol to obtain 5-ethoxy-5-hydroxy-4-oxo-4,5-dihydro-1H-pyrrolo[2,3-f]quinoline-2,7,9-tricarboxylic acid or salts thereof.

In an embodiment of the present disclosure, the salts of 4,5-dioxo-4,5-dihydro-1H-pyrrolo[2,3-f]quinoline-2,7,9-tricarboxylic acid (PQQ), 4,5-dioxo-4,5-dihydro-1H-pyrrolo[2,3-f]quinoline-2,7,9-tricarboxylic acid 2-allyl ester, 5-ethoxy-5-hydroxy-4-oxo-4,5-dihydro-1H-pyrrolo[2,3-f]quinoline-2,7,9-tricarboxylic acid and 5-hydroxy-6-(1,1,4-trioxo-1lambda*6*-1,2,5-thiadiazolidin-2-yl)-1H-indole-2-carboxylic acid are selected from a group comprising glucosamine, sodium, calcium and potassium or any combination thereof.

In another embodiment of the present disclosure, the coupling agent as disclosed above is selected from a group comprising 4-oxo-pent-2-enedioic acid dimethyl ester, copper acetate, 4-oxo-pent-2-enedioic acid diethyl ester, 4-oxo-pent-2-enedioic acid diisopropyl ester and 4-oxo-pent-2-enedioic acid di-tert-butyl ester, or any combination thereof; the oxidizing agent is ceric ammonium nitrate; and the hydrolysis is carried out in presence of base selected from a group comprising sodium carbonate, potassium carbonate, sodium hydroxide and lithium hydroxide, or any combination thereof.

In yet another embodiment of the present disclosure, the above process is carried out in presence of solvent selected from a group comprising water, tetrahydrofuran, methanol, benzyl alcohol, ethanol, tertiary butanol, ethyl acetate, acetonitrile, dichloromethane, N,N-dimethylformamide and acetic acid, or any combination thereof.

In still another embodiment of the present disclosure, the step (i) of the above described syntheses of 4,5-dioxo-4,5-dihydro-1H-pyrrolo[2,3-f]quinoline-2,7,9-tricarboxylic acid (PQQ), 4,5-dioxo-4,5-dihydro-1H-pyrrolo[2,3-f]quinoline-2,7,9-tricarboxylic acid 2-allyl ester and 5-ethoxy-5-hydroxy-4-oxo-4,5-dihydro-1H-pyrrolo[2,3-f]quinoline-2,7,9-tricarboxylic acid is carried out in presence of acid selected from a group comprising hydrochloric acid, trifluoroacetic acid and p-toluenesulfonic acid, formic acid, or any combination thereof.

In still another embodiment of the present disclosure, the above described syntheses of Formula IV, 4,5-dioxo-4,5-dihydro-1H-pyrrolo[2,3-f]quinoline-2,7,9-tricarboxylic acid (PQQ), 4,5-dioxo-4,5-dihydro-1H-pyrrolo[2,3-f]quinoline-2,7,9-tricarboxylic acid 2-allyl ester, 5-ethoxy-5-hydroxy-4-oxo-4,5-dihydro-1H-pyrrolo[2,3-f]quinoline-2,7,9-tricarboxylic acid and 5-hydroxy-6-(1,1,4-trioxo-1lambda*6*-1,2,5-thiadiazolidin-2-yl)-1H-indole-2-carboxylic acid is carried out at temperature ranging from about -20 °C to about 150 °C, preferably about -10 °C to about 100 °C and for a time period ranging from about 30 minutes to about 72 hours, preferably about 30 minutes to about 24 hours.

In still another embodiment of the present disclosure, the above described syntheses of Formula IV, 4,5-dioxo-4,5-dihydro-1H-pyrrolo[2,3-f]quinoline-2,7,9-tricarboxylic acid (PQQ), 4,5-dioxo-4,5-dihydro-1H-pyrrolo[2,3-f]quinoline-2,7,9-tricarboxylic acid 2-allyl ester, 5-ethoxy-5-hydroxy-4-oxo-4,5-dihydro-1H-pyrrolo[2,3-f]quinoline-2,7,9-tricarboxylic acid and 5-hydroxy-6-(1,1,4-trioxo-1lambda*6*-1,2,5-thiadiazolidin-2-yl)-1H-indole-2-carboxylic acid further comprise acts of precipitation or isolation or a combination thereof of the corresponding compound obtained; and wherein the isolation is carried out by acts selected from a group comprising addition of water, quenching, filtration and extraction, or any combination of acts in any order thereof.

In an object of the present disclosure, compounds of Formula I and tautomers, salts, isomers, analogs, or derivatives thereof are provided wherein, said compounds are employed in various applications such as synthesis of PQQ and salts thereof. The present disclosure also provides the synthesis procedure of said Formula I compounds.

In another object of the present disclosure, the compounds of Formula I serve as industrially useful starting compounds for the efficient synthesis of several pharmaceutically significant compounds such as:
1) 5-Hydroxy-6-(1,1,4-trioxo-1lambda*6*-1,2,5-thiadiazolidin-2-yl)-1H-indole-2-carboxylic acid (11) 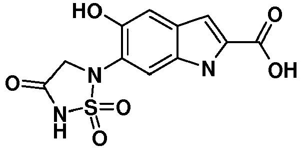
2) 4,5-Dioxo-4,5-dihydro-1H-pyrrolo[2,3-f]quinoline-2,7,9-tricarboxylic acid 2-allyl ester (16) 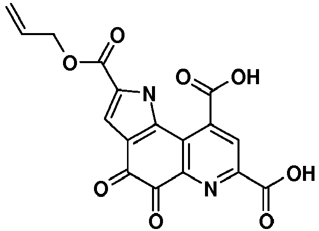
3) 5-Ethoxy-5-hydroxy-4-oxo-4,5-dihydro-1H-pyrrolo[2,3-f]quinoline-2,7,9-tricarboxylic acid (18) 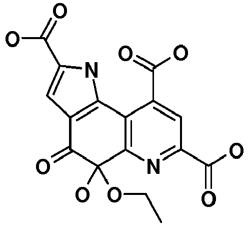 and
   corresponding salts thereof.

In an embodiment of the present disclosure, tautomeric forms of Formula I are also provided:

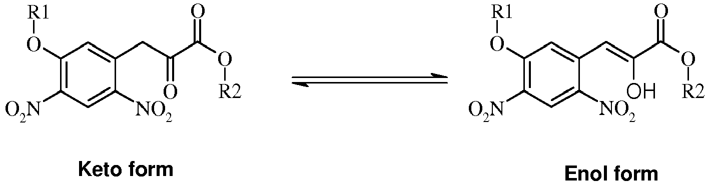

### Keto-enol tautomeric forms of Formula I

wherein, R1 and R2 are individually selected from a group comprising hydrogen, straight or branched chain C₁₋₈ alkyl, straight or branched chain C₁₋₈ alkenyl, straight or branched chain C₁₋₈ alkynyl, aralkyl, substituted aralkyl, alkaryl, substituted alkaryl, heteroaralkyl, substituted heteroaralkyl and wherein each of them may be optionally substituted,

In another object of the instant disclosure, synthesis of pyrroloquinoline quinone (PQQ) using Formula I [3-(5-substituted oxy-2, 4-dinitro-phenyl)-2-oxo-propionic acid ester] as intermediate compound is provided. The description also provides a process for the synthesis of salts of PQQ (such as sodium salt). More specifically, the synthesis of the PQQ using the intermediate compounds of Formula I is performed in only six steps to obtain a pure compound in less time and low cost. The instant synthesis of making PQQ is environmental friendly, safe and cost effective.

As described above, the compounds of Formula I are employed for the synthesis of pyrroloquinoline quinone (PQQ) and its salts thereof. In particular, the synthetic processes for the preparation of pyrroloquinoline quinone (PQQ) and its salts involve compounds of Formula I as intermediates. Further, the processes for the preparation of PQQ and the respective salts described in the present disclosure are cost-effective and efficient.

In an embodiment of the present disclosure, the various chemical compounds and intermediates as disclosed herein are as follows:

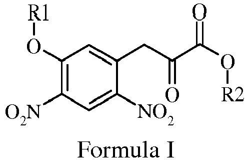

### [3-(5-substituted oxy-2,4-dinitro-phenyl)-2-oxo-propionic acid esters]

wherein, R1 and R2 are individually selected from a group comprising, but not limited to, hydrogen, straight or branched chain C₁₋₈ alkyl, straight or branched chain C₁₋₈ alkenyl, straight or branched chain C₁₋₈ alkynyl, aralkyl, substituted aralkyl, alkaryl, substituted alkaryl, heteroaralkyl, substituted heteroaralkyl and wherein each of them may be optionally substituted. 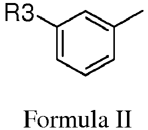
wherein, R3 is selected from a group comprising, but not limited to, fluoro, chloro, bromo, iodo, methanesulfonyloxy, toluenesulfonyloxy, trifluoromethanesulfonyloxy, alkyloxy, aryloxy, arolkyl oxy, heteroaryloxy and wherein each of them may be optionally substituted. 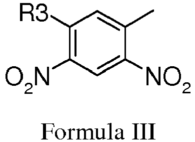 wherein, R3 is selected from a group comprising, but not limited to, fluoro, chloro, bromo, iodo, methanesulfonyloxy, toluenesulfonyloxy, trifluoromethanesulfonyloxy, alkyloxy, aryloxy, arolkyl oxy, heteroaryloxy and wherein each of them may be optionally substituted. 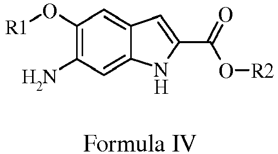 wherein, R1 and R2 are individually selected from a group comprising, but not limited to, hydrogen, straight or branched chain C₁₋₈ alkyl, straight or branched chain C₁₋₈ alkenyl, straight or branched chain C₁₋₈ alkynyl, aralkyl, substituted aralkyl, alkaryl, substituted alkaryl, heteroaralkyl, substituted heteroaralkyl and wherein each of them may be optionally substituted. 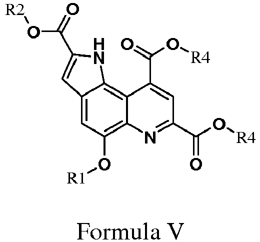 wherein, R1 and R2 are individually selected from a group comprising, but not limited to, hydrogen, straight or branched chain C₁₋₈ alkyl, straight or branched chain C₁₋₈ alkenyl, straight or branched chain C₁₋₈ alkynyl, aralkyl, substituted aralkyl, alkaryl, substituted alkaryl, heteroaralkyl, substituted heteroaralkyl and wherein each of them may be optionally substituted; and R4 is selected from group comprising, but not limited to, hydrogen, straight or branched chain C₁₋₈ alkyl, straight or branched chain C₁₋₈ alkenyl, straight or branched chain C₁₋₈ alkynyl, aralkyl, substituted aralkyl, heteroaralkyl, substituted heteroaralkyl and wherein each of them may be optionally substituted. 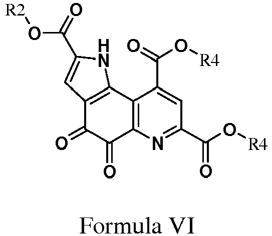 wherein, R2 is selected from a group comprising, but not limited to, hydrogen, straight or branched chain C₁₋₈ alkyl, straight or branched chain C₁₋₈ alkenyl, straight or branched chain C₁₋₈ alkynyl, aralkyl, substituted aralkyl, alkaryl, substituted alkaryl, heteroaralkyl, substituted heteroaralkyl and wherein each of them may be optionally substituted; and R4 is selected from group comprising, but not limited to, hydrogen, straight or branched chain C₁₋₈ alkyl, straight or branched chain C₁₋₈ alkenyl, straight or branched chain C₁₋₈ alkynyl, aralkyl, substituted aralkyl, heteroaralkyl, substituted heteroaralkyl and wherein each of them may be optionally substituted. 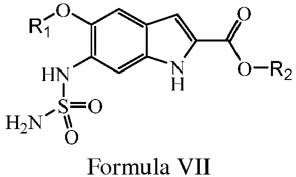 wherein, R1 and R2 are individually selected from a group comprising hydrogen, straight or branched chain C₁₋₈ alkyl, straight or branched chain C₁₋₈ alkenyl, straight or branched chain C₁₋₈ alkynyl, aralkyl, substituted aralkyl, alkaryl, substituted alkaryl, heteroaralkyl, substituted heteroaralkyl and wherein each of them may be optionally substituted; and R4 is selected from group comprising, but not limited to, hydrogen, straight or branched chain C₁₋₈ alkyl, straight or branched chain C₁₋₈ alkenyl, straight or branched chain C₁₋₈ alkynyl, aralkyl, substituted aralkyl, heteroaralkyl, substituted heteroaralkyl and wherein each of them may be optionally substituted.

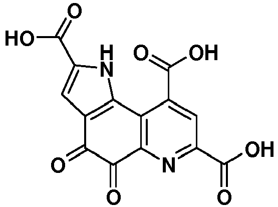

### Pyrroloquinoline quinone (PQQ)

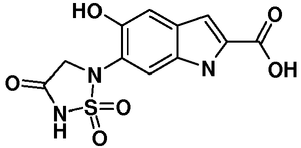

### 5-Hydroxy-6-(1,1,4-trioxo-1lambda*6*-1,2,5-thiadiazolidin-2-yl)-1H-indole-2-carboxylic acid

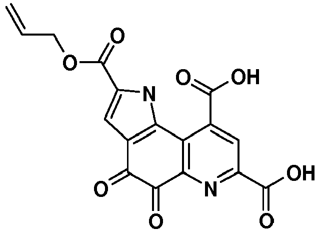

### 4,5-Dioxo-4,5-dihydro-1H-pyrrolo[2,3-f]quinoline-2,7,9-tricarboxylic acid 2-allyl ester

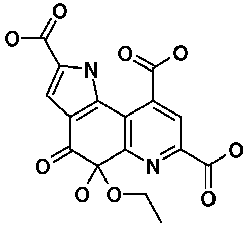

### 5-Ethoxy-5-hydroxy-4-oxo-4,5-dihydro-1H-pyrrolo[2,3-f]quinoline-2,7,9-tricarboxylic acid

In another embodiment of the present disclosure, the overall synthetic scheme for the preparation of pyrroloquinoline quinone (PQQ) comprises the following six steps:

### Step 1

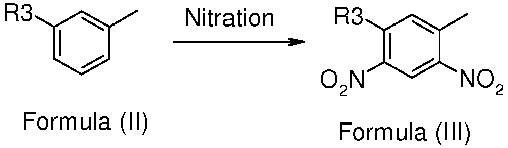

Compound of Formula III is prepared by the treatment of compound of Formula II with a suitable nitration mixture in presence of a suitable solvent at a temperature of about -10 °C to about 80 °C for a time period ranging from about 30 minutes to 48 hours. Preferably, the reaction is carried out at a temperature of about -10 °C to about 50 °C for a time period of about 30 minutes to about 2 hours.

In an embodiment of the present disclosure, suitable nitration mixture is selected from, but not limited to, a mixture of nitric acid and sulphuric acid, calcium nitrate and acetic acid, tert-butyl nitrite and the likes. Further, suitable solvent for the reaction is selected from, but not limited to, water, acetic acid and the likes. After the completion of reaction, compound of Formula III is isolated by suitable technique such as addition of the reaction mixture to water and filtration of the precipitated compound of Formula III.

Further, R3 of Formula II and Formula III is individually selected from a group comprising, but not limited to, fluoro, chloro, bromo, iodo, methanesulfonyloxy, toluenesulfonyloxy, trifluoromethanesulfonyloxy, alkyloxy, aryloxy, arolkyl oxy, heteroaryloxy and wherein each of them may be optionally substituted.

### Step 2

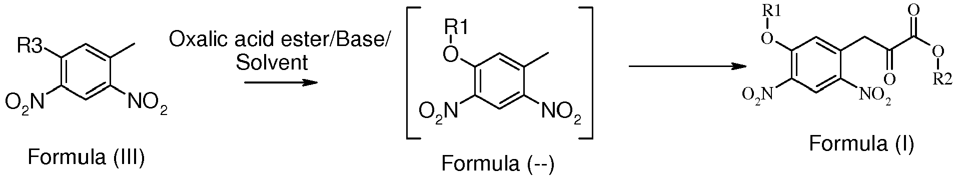

The compound of Formula I is directly synthesized by the reaction of compound of Formula III with suitable oxalic acid ester and a suitable base in the presence of solvent at a temperature of about -20 °C to about 100 °C for a time-period ranging from about 60 minutes to about 72 hours. The reaction is preferably carried out at a temperature of about 0 °C to about 40 °C and time-period of about one hour to about 36 hours.

In an embodiment of the present disclosure, suitable oxalic acid ester is selected from, but not limited to dimethyl oxalate, diethyl oxalate and the likes. Further, suitable base is selected from, but not limited to sodium methoxide, sodium ethoxide, potassium hydroxide potassium *t-*butoxide, sodium hydride, 1,8-diazabicyclo[5.4.0]undec-7-ene and the likes. Additionally, suitable solvent for the reaction is selected from, but not limited to methanol, benzyl alcohol, tertiary butanol, ethanol, tetrahydrofuran, dimethyl formamide and the likes.

In another embodiment, after the completion of reaction, compound of Formula I is isolated by suitable technique such as quenching the reaction mixture with acid, filtration, extraction with solvent or proceeded as such for the next step. Solvent for extraction of the product is selected from, but not limited to esters or halogenated solvents. Preferably, the solvent used is ethyl acetate or dichloromethane.

Further, R1 and R2 of Formula I are individually selected from a group comprising, but not limited to, hydrogen, straight or branched chain C₁₋₈ alkyl, straight or branched chain C₁₋₈ alkenyl, straight or branched chain C₁₋₈ alkynyl, aralkyl, substituted aralkyl, heteroaralkyl, substituted heteroaralkyl and wherein each of them may be optionally substituted.

### Step 3

Compound of Formula IV is prepared by the treatment of compound of Formula I with a suitable reducing agent in a suitable solvent at a temperature of about -10 °C to about 80 °C and for a time-period ranging from about 60 minutes to about 72 hours. The reaction is preferably carried out at a temperature of about 0 °C to about 50 °C and for a time-period of about one hour to about 16 hours.

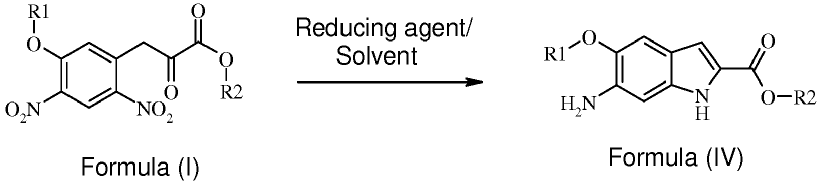

In an embodiment of the present disclosure, suitable reducing agent is selected from, but not limited to, palladium on carbon or raney-nickel under hydrogen pressure, palladium, sodium dithionite, iron/hydrochloric acid, zinc/acetic acid, tin chloride/hydrochloric acid and the likes. Suitable solvent for the reaction is selected from, but not limited to methanol, benzyl alcohol, ethanol, tertiary butanol, mixture of methanol and dichloromethane, N,N-Dimethylformamide, acetic acid and the likes, or any combinations thereof. In another embodiment, after the completion of reaction, compound of Formula IV is isolated by suitable technique such as filtration with solvent or proceeded as such for the next step. Further, solvent for filtration of the product is selected from, but not limited to ethanol, methanol or the likes.

Further, R1 and R2 of Formula IV are individually selected from a group comprising, but not limited to, hydrogen, straight or branched chain C₁₋₈ alkyl, straight or branched chain C₁₋₈ alkenyl, straight or branched chain C₁₋₈ alkynyl, aralkyl, substituted aralkyl, alkaryl, substituted alkaryl, heteroaralkyl, substituted heteroaralkyl and wherein each of them may be optionally substituted.

### Step 4

Compound of Formula V is prepared by coupling of compound Formula IV with suitable 4-oxo-pent-2-enedioic acid ester as a coupling agent in presence of suitable acid in a suitable solvent at a temperature of about -10 °C to about 80 °C and for time-period of about 60 minutes to 72 hours. The reaction is preferably carried out at a temperature of about 0 °C to about 50 °C and for a time-period ranging from about one hour to about 24 hours.

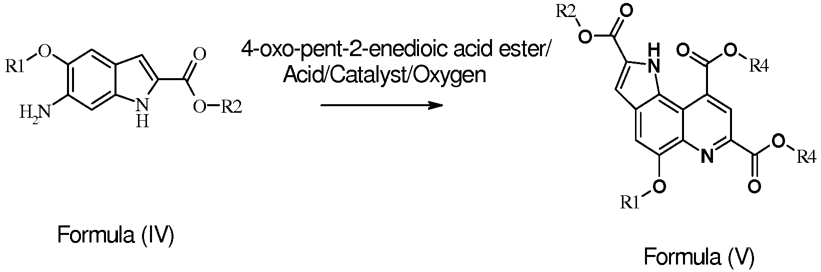

In an embodiment, suitable coupling agent is selected from, but not limited to 4-oxo-pent-2-enedioic acid dimethyl ester, copper acetate, 4-oxo-pent-2-enedioic acid diethyl ester, and the likes. Further, suitable solvent for the reaction is selected from, but not limited to dichloromethane, tetrahydrofuran, ethyl acetate, acetonitrile and the likes. Suitable acid for the reaction is selected from hydrochloric acid, trifluoroacetic acid, p-toluenesulfonic acid, formic acid and the likes. In another embodiment, after the completion of reaction, compound of Formula V is isolated by suitable technique such as filtration with solvent or proceeded as such for the next step. Further, solvent for filtration of the product is selected from, but not limited to ethanol, methanol and the likes.

Further, under Formula V, R1 and R2 are individually selected from a group comprising, but not limited to, hydrogen, straight or branched chain C₁₋₈ alkyl, straight or branched chain C₁₋₈ alkenyl, straight or branched chain C₁₋₈ alkynyl, aralkyl, substituted aralkyl, alkaryl, substituted alkaryl, heteroaralkyl, substituted heteroaralkyl and wherein each of them may be optionally substituted; and R4 is selected from group comprising, but not limited to, hydrogen, straight or branched chain C₁₋₈ alkyl, straight or branched chain C₁₋₈ alkenyl, straight or branched chain C₁₋₈ alkynyl, aralkyl, substituted aralkyl, heteroaralkyl, substituted heteroaralkyl and wherein each of them may be optionally substituted.

### Step 5

Compound of Formula VI is prepared by oxidative demethylation of compound of Formula V with suitable oxidizing agent in a suitable solvent at a temperature of about -10 °C to about 80 °C for a time-period of about 30 minutes to about 48 hours. The reaction is preferably carried out at a temperature of about -10 °C to about 0 °C for time-period of about one hour to about 4 hours.

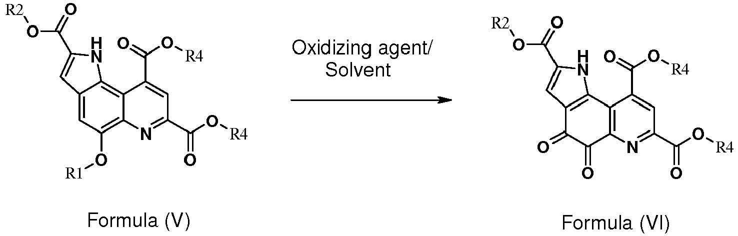

In an embodiment, suitable oxidizing agent is selected from, but not limited to ceric ammonium nitrate or the likes. Suitable solvent for the reaction is selected from, but not limited to acetonitrile, ethyl acetate, dichloromethane, tetrahydrofuran, a mixture of acetonitrile and dichloromethane and the likes. After the completion of reaction, compound of Formula VI is isolated by suitable technique such as filtration with solvent, extraction with solvent or proceeded as such for the next step. Further, solvent for extraction of the product is selected from, but not limited to dichloromethane, chloroform and the likes. Additionally, solvent for filtration of the product is selected from, but not limited to acetonitrile, ethyl acetate, water and the likes.

Further, under Formula VI, R2 is selected from a group comprising, but not limited to, hydrogen, straight or branched chain C₁₋₈ alkyl, straight or branched chain C₁₋₈ alkenyl, straight or branched chain C₁₋₈ alkynyl, aralkyl, substituted aralkyl, alkaryl, substituted alkaryl, heteroaralkyl, substituted heteroaralkyl and wherein each of them may be optionally substituted; and R4 is selected from group comprising, but not limited to, hydrogen, straight or branched chain C₁₋₈ alkyl, straight or branched chain C₁₋₈ alkenyl, straight or branched chain C₁₋₈ alkynyl, aralkyl, substituted aralkyl, heteroaralkyl, substituted heteroaralkyl and wherein each of them may be optionally substituted.

### Step 6

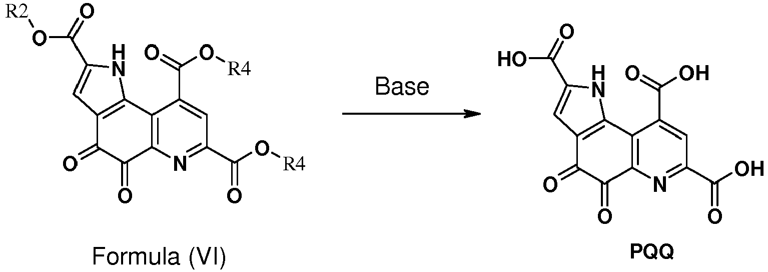

Compound pyrroloquinoline quinone (PQQ) is prepared by base hydrolysis of compound of Formula VI with suitable base in a suitable solvent at a temperature of about 10 °C to about 150 °C for a time-period ranging from about 60 minutes to about 72 hours. The reaction is preferably carried out at a temperature of about 10 °C to about 100 °C and for a time-period of about one hour to about 24 hours.

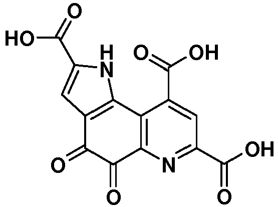

### 4,5-Dioxo-4,5-dihydro-1H-pyrrolo[2,3-f]quinoline-2,7,9-tricarboxylic acid (PQQ)

In an embodiment, suitable base is selected from, but not limited to sodium hydroxide, potassium hydroxide, sodium carbonate, potassium carbonate and the likes. Suitable solvent for the reaction is selected from, but not limited to water, tetrahydrofuran, methanol, benzyl alcohol, tertiary butanol, ethanol, a mixture of water and ethanol and the likes. After the completion of reaction, compound PQQ is precipitated by quenching the reaction mass with suitable acid, such as hydrochloric acid, hydrobromic acid, phosphoric acid and the likes. The precipitated PQQ is isolated by suitable techniques such as filtration with solvent. Solvent for filtration of the precipitated PQQ is selected from, but not limited to acetonitrile, ethyl acetate, water and the likes.

### Step 6' (synthesizing salts of PQQ)

PQQ salts are prepared by base hydrolysis of compound of Formula VI with suitable base (Sodium hydroxide, Potassium hydroxide, Potassium carbonate, Lithium hydroxide) in a suitable solvent system (water, Ethanol, methanol or mixuture of the solvents) and under appropriate reaction conditions. For instance, compound pyrroloquinoline quinone (PQQ) disodium salt is prepared by base hydrolysis of compound of Formula VI with a solution of sodium carbonate or sodium hydroxide suitable base in a suitable solvent at a temperature of about 10 °C to about 150 °C for a time-period ranging from about 60 minutes to about 72 hours. The reaction is preferably carried out at a temperature of about 10 °C to about 100 °C and for a time-period of about one hour to about 24 hours. 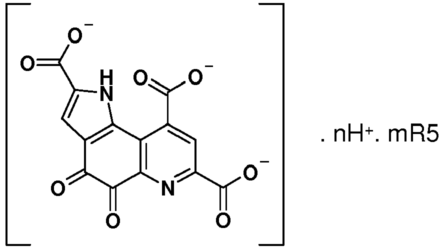
wherein n=1, m=2 and R5 is Na⁺

### 4,5-Dioxo-4,5-dihydro-1H-pyrrolo[2,3-f]quinoline-2,7,9-tricarboxylic acid (PQQ) disodium

In an embodiment, suitable base is selected from, but not limited to sodium carbonate, potassium carbonate, sodium hydroxide and the likes. Suitable solvent for the reaction is selected from, but not limited to water, tetrahydrofuran, methanol, benzyl alcohol, tertiary butanol, ethanol, a mixture of water and ethanol and the likes. After the completion of reaction, compound PQQ disodium is precipitated by adjusting pH (pH=3.0 to 3.5) of the reaction mass with suitable acid such as hydrochloric acid, hydrobromic acid, phosphoric acid and the likes. The precipitated PQQ disodium is isolated by suitable techniques, such as filtration with solvent. Solvent for filtration of the precipitated PQQ disodium is selected from, but not limited to acetonitrile, water and the likes.

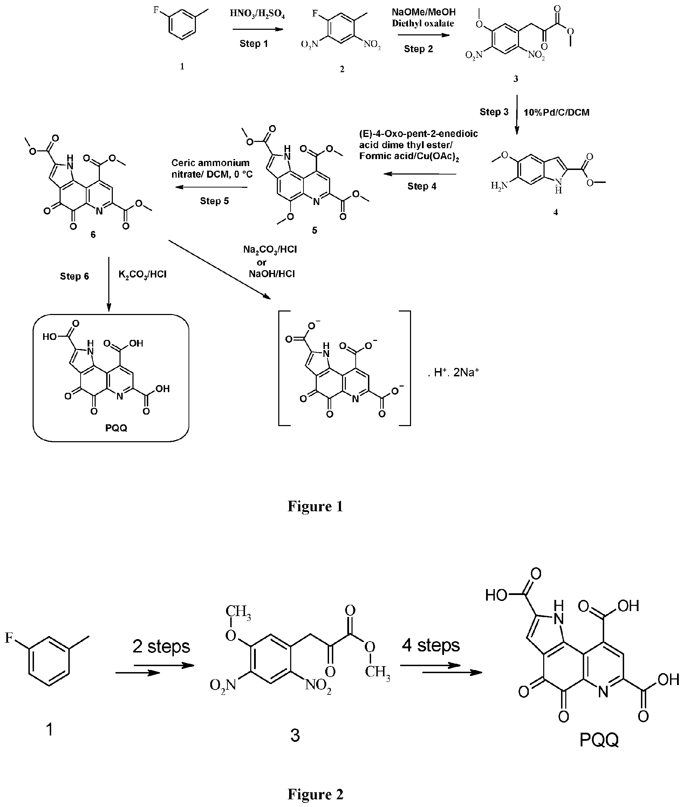

In one embodiment of the present disclosure, the truncated method of synthesis of PQQ using intermediate compound 3: (3-(5-substituted Oxy-2, 4-dinitro-phenyl)-2-oxo-propionic acid ester) is provided in Figure 2. In another embodiment, the intermediate compound 3 is synthesized in two steps using compound 1 (3-fluoro toluene).

A series of 6 steps are used to synthesize PQQ as described above. A synthesis scheme using specific compounds/intermediates is provided in Figure 1. In one embodiment, the starting material for 'step 1' is 1-flouro-3-methyl-benzene at specific concentration which is added to a nitration mixture at a specific concentration and temperature and at a specific time in reaction 1. The reaction mixture is cooled to precipitate the product which is further filtered, dried under vacuum and washed with hexane to remove the impurities and to obtain compound 2.

In another embodiment, compound 2 is suspended in a solvent (such as methanol) and reacted with sodium methoxide and diethyl oxalate at a special temperature and special concentration to yield intermediate compound annotated as compound 3 in the instant specification. In one exemplary example, the chemical name for the compound 3 is '3-(5-methoxy-2,4-dinitro-phenyl)-2-oxo-propionic acid methyl ester'.

In one embodiment, compound 3 is reacted with 10% Pd/C in a mixture of dicholoromethane and methanol in a specific ratio under a hydrogen atmosphere to obtain compound 4.

In another embodiment, as described in detail in the detailed description section, a series of reactions are performed with specific chemical and compound 4 dissolved in dicholoromethane to obtain compound 5. In another embodiment, compound 6 is obtained by dissolving compound 5 in acetonitrile and reacting with a series of solutions having specific concentration and time period, as stated in detailed description of this application. In an exemplary embodiment, compound 3 is employed as a starting material to make compound 4, compound 5 and compound 6 thus, synthesizing PQQ in the sixth step.

In yet another embodiment, sodium salt of PQQ is made by using sodium carbonate and/or sodium hydroxide.

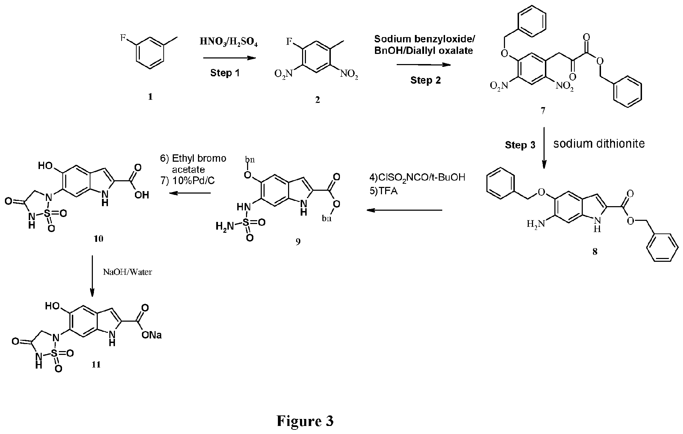

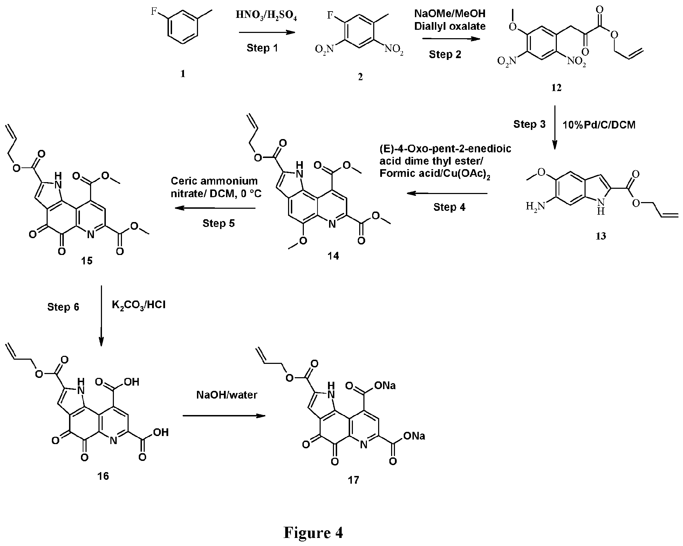

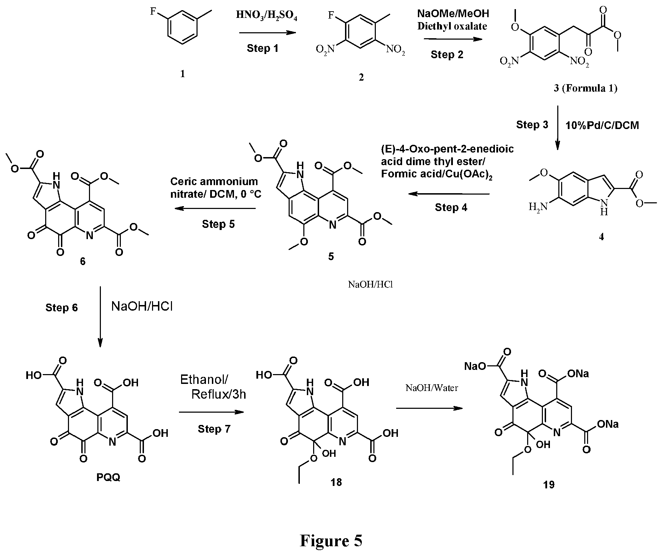

In an exemplary embodiment, Formula I is used as an intermediate to synthesize compounds, such as, 5-hydroxy-6-(1,1,4-trioxo-1lambda*6*-1,2,5-thiadiazolidin-2-yl)-1H-indole-2-carboxylic acid, 4,5-dioxo-4,5-dihydro-1H-pyrrolo[2,3-f]quinoline-2,7,9-tricarboxylic acid 2-allyl ester, 5-ethoxy-5-hydroxy-4-oxo-4,5-dihydro-1H-pyrrolo[2,3-f]quinoline-2,7,9-tricarboxylic acid and salts thereof. The synthesis of said compounds are depicted in Figures 3, 4 and 5 respectively.

The compound, processes and methods of making the intermediary compounds, PQQ, 5-hydroxy-6-(1,1,4-trioxo-1lambda*6*-1,2,5-thiadiazolidin-2-yl)-1H-indole-2-carboxylic acid, 4,5-dioxo-4,5-dihydro-1H-pyrrolo[2,3-f]quinoline-2,7,9-tricarboxylic acid 2-allyl ester, 5-ethoxy-5-hydroxy-4-oxo-4,5-dihydro-1H-pyrrolo[2,3-f]quinoline-2,7,9-tricarboxylic acid and the corresponding salts disclosed herein may be implemented in any means for achieving various aspects, and may be executed to produce the said compounds in large scale. Other features will be apparent from the accompanying drawings and from the detailed description.

The present disclosure is further elaborated with the help of following examples. However, the examples should not be construed to limit the scope of the present disclosure.

### Examples

### Example 1: Preparation of 1-fluoro-5-methyl-2,4-dinitro-benzene [Formula III, wherein R3 is F]

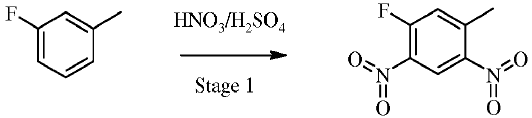

Nitric acid 67% (about 250 mL, 5.6 mol) is charged to a 3-necked round bottomed flask equipped with mechanical stirrer at a temperature of about 25-30 °C. Concentrated sulfuric acid (about 360 mL. 6.75 mol) is thereafter added drop wise at about 0 °C over a period of about 30 minutes. To the obtained nitration mixture, 1-fluoro-3-methyl-benzene [Formula II, wherein R3 is F] (about 100 mL, 0.9 mol) is added drop wise at a temperature ranging about 10-20 °C over a period of about 20 minutes.

The resulting reaction mixture is allowed to stir at a temperature ranging about 20-25 °C over a period of about 30 minutes. Completion of the reaction is monitored (10% ethyl acetate in petroleum ether as solvent system) by thin-layer chromatography (TLC). Thereafter, the reaction mixture is poured into crushed ice (about 3.0 L) to precipitate the product. The precipitated product is filtered and dried under house vacuum (for about 2 hours). The product obtained is suspended in n-heptane (about 1.0 L) for a wash and filtered. The same hexane wash (about 2X1.0 L) is repeated about twice to remove non polar impurities. The product [1-fluoro-5-methyl-2,4-dinitro-benzene] (about 129 g, 71%) obtained upon filtration is taken to next step.
**¹H NMR (DMSO-d₆, 300 MHz):** 2.63 (s, 3H), 7.86 (d, *J*=12.0 Hz, 1H), 8.74 (d, *J*=6.9 Hz, 1H). **LC-MS (ESI):** 199.0 (M-H).

### Example 2

### Example 2A: Preparation of 3-(5-methoxy-2,4-dinitro-phenyl)-2-oxo-propionic acid methyl ester [Formula I, wherein R1 is -CH₃ and R2 is -CH₃]

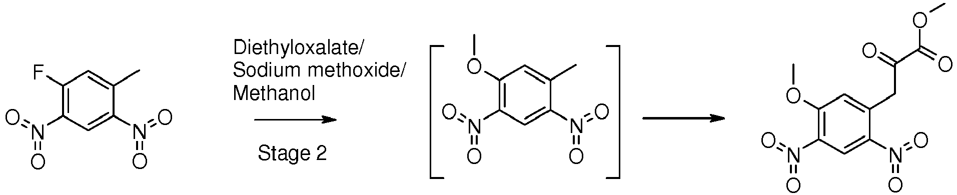

To a suspension of 1-fluoro-5-methyl-2,4-dinitro-benzene (about 100.0g, 0.5 mol) in methanol (about 1.5 L), sodium methoxide (about 108 g, 1.9 mol) and diethyl oxalate (about 136 mL, 1.0 mol) are added at a temperature of about -10°C. The resulting orange color suspension is slowly allowed to reach a temperature of about 15-20 °C in about 2 hours and continuous stirring is carried out at a temperature of about 15-20 °C over a period of about 16 hours. The reaction mixture is quenched with 1.5 N HCl (about 200 mL), the precipitated solid obtained is filtered and dried under house vacuum to obtain the product [3-(5-methoxy-2,4-dinitro-phenyl)-2-oxo-propionic acid methyl ester] as pale yellow solid (about 125 g, 85%).
**¹H NMR (CDCl₃, 300 MHz):** 3.98 (s, 3H), 4.11 (s, 3H), 4.65 (s, 2H), 7.00 (s, 1H), 8.84 (s, 1H); **LC-MS (ESI):** 297.0 (M-H);
HPLC purity: 99.12%
Melting Range: 142.7°C-143.2°C
IR (KBr, Cm⁻¹): 1732, 1649, 1614, 1588, 1520, 1355, 1282, 1070 and 840

### HPLC method of analysis:

### 1.1 Chromatographic conditions

| | |
|---|---|
| Column | : INERTSIL C8, 150mm X 4.6 mm, 3µm |
| Mobile phase A | : 20 mM Ammonium dihydrogen phosphate in Water(pH-2.1), |
| Mobile phase B | : Acetonitrile |
| Flow rate | : 0.8 mL/min. |
| Injection Volume | : 5 µL |
| Column Temperature | : 25°C |
| Detection Wavelength | : 210 nm |
| Diluent | : Acetonitrile 100% |
| Run Time | : 30 minutes |

### 1.2 Gradient Programming

| **Time in Minutes** | **% of Mobile phase A** | **% of Mobile phase** B |
|---|---|---|
| 0 | 98 | 02 |
| 15 | 20 | 80 |
| 22 | 20 | 80 |
| 25 | 98 | 02 |
| 30 | 98 | 02 |

### 1.3 Mobile phase preparation

- Preparation of mobile phase A (20 mM Ammonium dihydrogen phosphate in Water (pH-2.1) Weigh accurately about 2.3g of ammonium dihydrogen phasphate into 1000mL clean and dry one liter mobile phase bottle containing 1000mL water and adjust the pH to 2.1 with orthophosphoric acid. Sonicate and filter through 0.45µm membrane filter and degas.
- Preparation of mobile phase B (Acetonitrile)

Transfer 1000mL of Acetonitrile into a clean and dry one liter mobile phase bottle.

### 1.4 Sample Preparation

Weigh accurately about 20mg of sample and transfer to 100mL volumetric flask, add 40mL of diluent to dissolve and make up to volume with the same diluent.

### Example 2B : Preparation of 3-(5-methoxy-2,4-dinitro-phenyl)-2-oxo-propionic acid methyl ester [Formula I sodium salt, wherein R1 is -CH₃ and R2 is -CH₃]

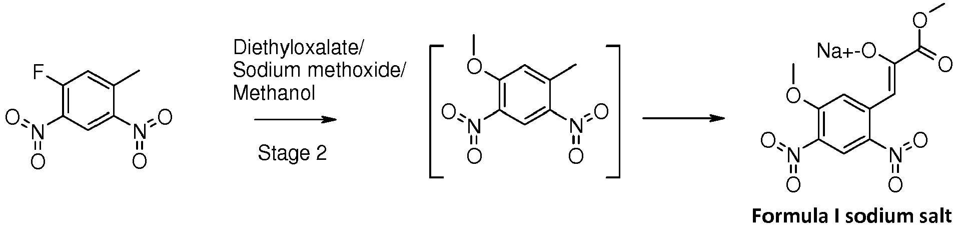

To a suspension of 1-fluoro-5-methyl-2,4-dinitro-benzene (about 4.0g, 16.0 mmol) in methanol (about 50 mL), sodium methoxide (about 4.3 g, 76 mmol) and diethyl oxalate (about 5.4 mL, 37 mmol) are added at a temperature of about 0 °C. The resulting orange color suspension is slowly allowed to reach a temperature of about 15-20 °C in about 2 hours and continuous stirring is carried out at a temperature of about 15-20 °C over a period of about 16 hours. Completion of the reaction was monitored by TLC. The precipitated solid obtained is filtered and dried under house vacuum to obtain the product [sodium salt of 2-Hydroxy-3-(5-methoxy-2,4-dinitro-phenyl)-acrylic acid methyl ester] as brown solid (about 5.0 g, 86%).
**¹H NMR (DMSO-d₆, 400 MHz):** 3.66 (s, 3H), 3.87 (s, 3H), 6.87 (s, 1H), 8.66 (s, 1H), 9.12 (s, 1H); **LC-MS (ESI):** 297.0 (M-H);
HPLC purity: 95 %
Melting Range: 250.7°C-259.8°C
IR (KBr, Cm⁻¹): 3400, 1731, 1653, 1614, 1547, 1331, 1270, 1208 and 780

### Example 2C: Preparation of 3-(5-ethoxy-2,4-dinitro-phenyl)-2-oxo-propionic acid ethyl ester [Formula I, wherein R1 is -CH₂CH₃ and R2 is - CH₂CH₃]

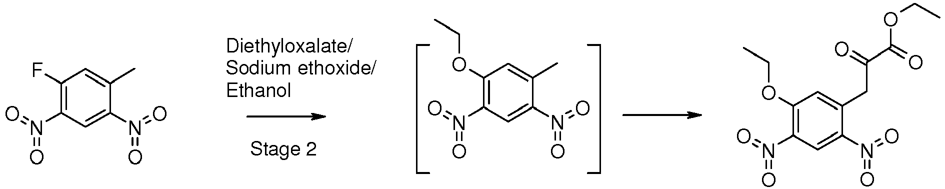

To a suspension of 1-fluoro-5-methyl-2,4-dinitro-benzene (about 100.0 g, 0.5 mol) in ethanol (about 1.5 L), sodium ethoxide (about 136 g, 1.9 mol) and diethyl oxalate (about 136 mL, 1.0 mol) are added at a temperature of about 0°C. The resulting orange color suspension is slowly allowed to reach a temperature of about 15-20 °C in about 2 hours and continuous stirring is carried out at a temperature of about 15-20 °C over a period of about 24 hours. The reaction mixture is quenched with 1.5 N HCl (about 200 mL), the precipitated solid obtained is filtered and dried under house vacuum to obtain the product [3-(5-ethoxy-2,4-dinitro-phenyl)-2-oxo-propionic acid ethyl ester] as pale yellow solid (about 126 g, 76%).
**¹H NMR (DMSO-d₆, 400 MHz):** 1.43 (t, *J* = 7.2 Hz, 3H), 1.55 (t, *J* = 6.8 Hz, 3H), 4.32 (q, *J* = 6.8 Hz, 2H), 4.42 (q, *J* = 7.2 Hz, 2H), 4.62 (s, 2H), 6.96 (s, 1H), 8.81 (s, 1H); **LC-MS (ESI):** 325.0 (M-H);
HPLC purity: 98 %

### Example 2D: Preparation of 3-(5-methoxy-2,4-dinitro-phenyl)-2-oxo-propionic acid ethyl ester [Formula I, wherein R1 is -CH₃ and R2 is - CH₂CH₃]

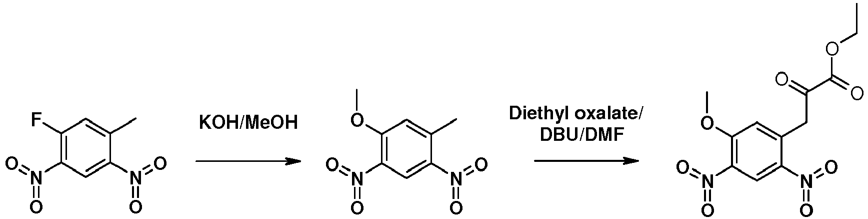

To a suspension of 1-fluoro-5-methyl-2,4-dinitro-benzene (100 g, 0.5 mol) in methanol (0.5 L) was added KOH (56 g, 0.1 mmol) in methanol (0.5 mL) drop wise at 25-30 °C. Then the resulting suspension was stirred at room temperature over a period of 30 minutes. The reaction mixture was quenched with water at 0-5 °C, the reaction mixture was filtered and dried under vacuum to obtain 1-methoxy-5-methyl-2,4-dinitro-benzene as off-white solid (101 g, 95%).
¹**H NMR** (**DMSO**-**d6**, **300 MHz):** 2.67 (s, 3H), 4.05 (s, 3H), 7.52 (s, 1H), 8.65 (s, 1H); **LC-MS (ESI):** 211.0 (M-H);

To a solution of 1-methoxy-5-methyl-2,4-dinitro-benzene (100 g, 0.47 mol) in N,N-dimethylformamide (500 mL) were added 1,8-diazabicyclo[5.4.0]undec-7-ene (143 g, 0.94 mol) and diethyl oxalate (129 mL, 0.94 mol) at -10 °C. The reaction mixture was stirred at 25-30 °C over a period of 16 h. Then reaction mixture was quenched with 1.5 N HCl (pH=2.0) and diluted with dichloromethane (1.5 L). The organic layer was separated and washed with brine, dried over sodium sulphate and evaporated under reduced pressure. The residue obtained upon evaporation of the solvent was washed methyl tert-butyl ether to yield 3-(5-methoxy-2,4-dinitro-phenyl)-2-oxo-propionic acid ethyl ester as a yellow solid (117 g, 80%).
**¹H NMR (DMSO-d6, 300 MHz):** 1.28-1.34 (m, 3H), 4.02 & 4.04 (2s, 3H), 4.27-4.34 (m, 2H), 4.74 (s, 1H), 6.83 (s, 0.5 H), 7.57 (s, 0.5 H), 8.05 (s, 0.5 H), 8.63 (s, 0.5 H), 8.75 (s, 0.5H), 10.83 (bs, 0.5H);
**LC-MS (ESI):** 311.0 (M-H);

### Example 3

### Example 3A: Preparation of 6-amino-5-methoxy-1H-indole-2-carboxylic acid methyl ester [Formula IV, wherein R1 is -CH₃ and R2 is -CH₃]

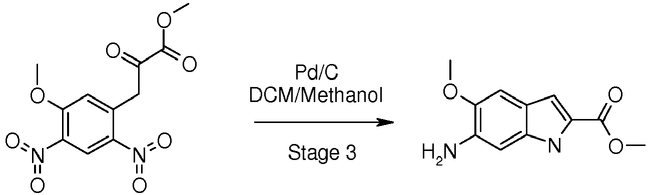

To a solution of 3-(5-methoxy-2,4-dinitro-phenyl)-2-oxo-propionic acid methyl ester (about 100.0g, 0.32 mol) in a mixture of dichloromethane and methanol (about 1.5 L), 10% Pd/C (about 30 g, 50% wet) is added at about 25 °C. The reaction mixture is stirred under hydrogen atmosphere at a temperature of about 25 °C over a period of about 4 hours. The resulting reaction mixture is filtered through celite pad and concentrated under reduced pressure to yield the product [6-amino-5-methoxy-1H-indole-2-carboxylic acid methyl ester] as yellow color solid (about 59 g, 80%).
**¹H NMR (DMSO-d₆, 300 MHz):** 3.78 (s, 3H), 3.79 (s, 3H), 4.99 (bs, 2H), 6.62 (s, 1H), 6.90 (s, 1H), 6.92 (s, 1H), 11.90 (s, 1H); **LC-MS (ESI):** 221.1; Purity by HPLC: 98%

### Example 3B: Preparation of 6-amino-5-methoxy-1H-indole-2-carboxylic acid methyl ester [Formula IV, wherein R1 is -CH₃ and R2 is -CH₃]

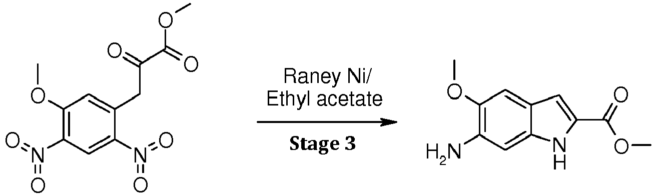

To a solution of 3-(5-methoxy-2,4-dinitro-phenyl)-2-oxo-propionic acid methyl ester (1. 0 kg, 1 eq, 3.353 moles) in ethyl acetate (20 L, 20 vol) was added Raney Nickel (1.0Kg,100%), which is pre washed with methanol and ethyl acetate. Stirred the reaction mixture at about 25 to 35 °C with 2 kg/cm² H₂ pressure for about 6hours. Completion of the reaction was ministered by HPLC. Filtered the reaction mass through celite pad, concentrated under reduced pressure to two volume and added hexane (6L) to precipitate the product. Filtered the precipitate and dried at about 45 to 50 °C for 8h (600 g, 80%).
**¹H NMR** (**DMSO-d₆, 300 MHz):** 3.78 (s, 3H), 3.79 (s, 3H), 4.99 (bs, 2H), 6.62 (s, 1H), 6.90 (s, 1H), 6.92 (s, 1H), 11.90 (s, 1H); **LC-MS (ESI):** 221.1; Purity by HPLC: 97.5%

### Example 4: Preparation of 5-methoxy-1H-pyrrolo[2,3-f]quinoline-2,7,9-tricarboxylic acid trimethyl ester [Formula V, wherein R1 is -CH₃ and R2 is -CH₃ and R4 is CH₃]

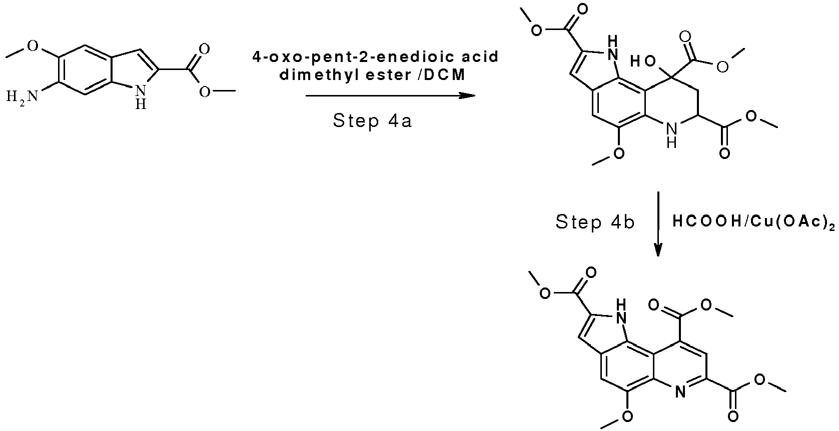

To a solution of 6-amino-5-methoxy-1H-indole-2-carboxylic acid methyl ester (about 264.0g, 1.18mol) in dichloromethane (about 2.64 L), 4-oxo-pent-2-enedioic acid dimethyl ester (about 227.0 g, 1.3 mol) is added at a temperature of about 25 °C. The reaction mixture is allowed to stir at about 25 °C over a period of about 16 hours. Then concentrated the reaction mixture under reduced pressure to minimum volume (500 mL) and precipitated reaction mass with hexane and filtered.

The precipitate obtained upon filtration was dissolved in formic acid (1.2 L), copper(II)acetate monohydrate (119 g, 0.59 mol) was added to the reaction mass allowed to stir at about 25 °C for a period of about 48 hours. Slowly added the reaction mass to water (2.5 L) at 15 to 20 °C and filtered the solids. The solid obtained was washed with mixture of methanol (2.5 L) and aqueous ammonia solution (300 mL) and dried for 12h at 45 to 50 °C under reduced pressure to obtain the product [5-methoxy-1H-pyrrolo[2,3-f]quinoline-2,7,9-tricarboxylic acid trimethyl ester] as a pale yellow solid (about 350 g, 80%).
**¹H NMR (DMSO-d₆, 300 MHz):** 3.94 (s, 3H), 4.00 (s, 3H), 4.02 (s, 3H), 4.11 (s, 2H), 7.32 (s, 1H), 7.56 (s, 1H), 8.69 (s, 1H), 12.01 (s, 1H); **LC-MS (ESI):** 371.0 (M-H).

### Example 5

### Example 5A: Preparation of 4,5-dioxo-4,5-dihydro-1H-pyrrolo[2,3-f]quinoline-2,7,9-tricarboxylic acid trimethyl ester [Formula VI, wherein R2 is -CH₃ and R4 is -CH₃]

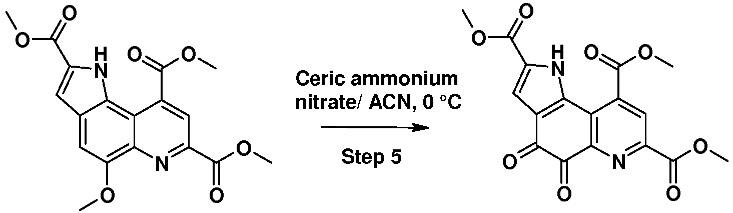

To a suspension of 5-methoxy-1H-pyrrolo[2,3-f]quinoline-2,7,9-tricarboxylic acid trimethyl ester (about 250g, 0.67 mol) in acetonitrile (ACN) (about 7.5 L), a solution of ceric ammonium nitrate (about 1.83 kg, 3.35 mol) in about 2.25 L of water is added over a period of about 30 minutes at about -5 °C. The reaction mixture is stirred at about -5°C over a period of about 1.5 hours. Thereafter, resulting reaction mass is filtered, washed with water and dried to obtain the product [4,5-dioxo-4,5-dihydro-1H-pyrrolo[2,3-f]quinoline-2,7,9-tricarboxylic acid trimethyl ester] as a bright orange solid (about 145 g, 58%).
**¹H NMR (DMSO-d₆, 300 MHz):** 3.90 (s, 3H), 3.97 (s, 3H), 4.05 (s, 3H), 7.29 (d, *J*=1.8 Hz, 1H), 8.56 (s, 1H), 12.52 (s, 1H); **LC-MS (ESI):** 373.0 (M-H).

### Example 5B: Preparation of 4, 5-dioxo-4,5-dihydro-1H-pyrrolo[2,3-f]quinoline-2,7,9-tricarboxylic acid trimethyl ester [Formula VI, wherein R2 is -CH₃ and R4 is -CH₃]

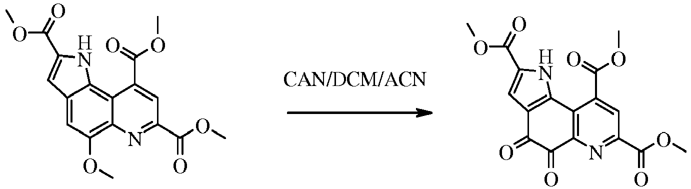

To a solution of 5-methoxy-1H-pyrrolo[2,3-f]quinoline-2,7,9-tricarboxylic acid trimethyl ester (1.0 kg, 1 eq, 2.686 moles) in acetonitrile (10 L) and DCM (10 L) was added solution of ceric ammonium nitrate (CAN) (5.89 kg, 4.0 eq, 10.74 moles, dissolved in 6 L water) slowly at about - 25 to -20 °C and stirred at same temperature for about 1hour. Diluted the reaction mass with DCM (30L, 30 vol). Added water (10L, 10 vol) to the reaction mass and separated the layers. Washed the organic layer twice with 1.5N HCl solution (5L, 5 vol) and with water (5L, 5 vol). The organic layer was dried over sodium sulphate and concentrated under reduced pressure. The residue was suspended with ethyl acetate (4L, 4vol) and filtered and dried at 45 to 50 °C for 8h under reduced pressure to obtain product as bright orange solid (550 g, 55%)
**¹H NMR (DMSO-d₆, 300 MHz):** 3.90 (s, 3H), 3.97 (s, 3H), 4.05 (s, 3H), 7.29 (d, *J*=1.8 Hz, 1H), 8.56 (s, 1H), 12.52 (s, 1H); **LC-MS (ESI):** 373.0 (M-H).

### Example 6: Preparation of 4,5-dioxo-4,5-dihydro-1H-pyrrolo[2,3-f]quinoline-2,7,9-tricarboxylic acid (PQQ)

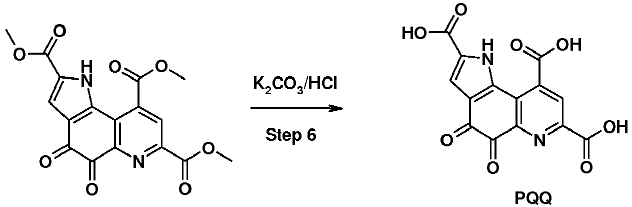

To a solution of potassium carbonate (about 439 g, 3.18 mol) in water (about 4.5 L), 4,5-dioxo-4,5-dihydro-1H-pyrrolo[2,3-f]quinoline-2,7,9-tricarboxylic acid trimethyl ester (about 200 g, 0.53 mol) is added at about 25 °C. The reaction mixture is allowed to stir at about 80 °C over a period of about 12 hours. Completion of reaction is monitored by HPLC. Thereafter, the reaction mixture is acidified (pH: 2.5) with 1N hydrochloric acid to precipitate the product [4,5-dioxo-4,5-dihydro-1H-pyrrolo[2,3-f]quinoline-2,7,9-tricarboxylic acid] as dark red solid (about 160 g, 91%).
**¹H NMR (DMSO-d₆, 300 MHz):** 7.21 (s, 1H), 8.60 (s, 1H), 13.42 (s, 1H); **LC-MS** (**ESI**)**:** 329 (M-H), **Purity by HPLC:** 98%

### HPLC method of Analysis:

### 1.1 Method outline

This method is applicable for the determination of percentage purity (area %) by High performance liquid chromatography. This method uses reverse phase HPLC.

### 1.2 Chromatographic conditions

| | |
|---|---|
| Column | : Atlantis C18, 5µm, 250mm x 4.6 mm |
| Mobile phase A | : 0.1% Trifluoroacetic acid in water |
| Mobile phase B | : Methanol |
| Flow rate | : 1.0 mL/min. |
| Injection Volume | : 20 µL |
| Column Temperature | : 25°C |
| Detection Wavelength | : 250 nm |
| Diluent | : Water:Acetonitrile: :80:20 |
| Run Time | : 40 minutes |

### 1.3 Gradient Programming

| **Time in Minutes** | **% of Mobile phase A** | **% of Mobile phase B** |
|---|---|---|
| 00 | 90 | 10 |
| 05 | 90 | 10 |
| 25 | 25 | 75 |
| 30 | 15 | 85 |
| 34 | 15 | 85 |
| 35 | 90 | 10 |
| 40 | 90 | 10 |

### 1.4 Mobile phase preparation

### • Preparation of mobile phase A (0.1%TFA in water)

Transfer 1 mL of Trifluoroacetic acid into 1000mL clean and dry one liter mobile phase containing 1000mL water. Sonicate and filter through 0.45µm membrane filter and degas.

### • Preparation of mobile phase B (Methanol)

Transfer 1000mL of Methanol into a clean and dry one liter mobile phase bottle.

### • Preparation of Diluent.

Transfer 400mL of water and 100mL acetonitrile into clean and dry mobile phase bottle. Shake vigorously to mix and sonicate.

### 1.5 Sample Preparation

Weigh accurately about 50mg of sample and transfer to 100mL volumetric flask, add 40mL of diluent to dissolve and make up to volume with the same diluent.

### Example 7:

### (A) Preparation of 4,5-Dioxo-4,5-dihydro-1H-pyrrolo[2,3-f]quinoline-2,7,9-tricarboxylic acid disodium (PQQ.2Na)

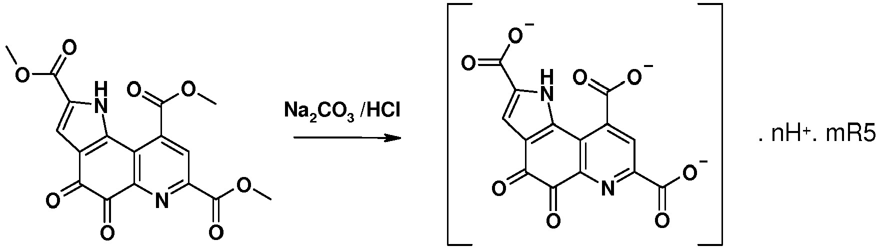

Where in n=1, m=2 and R5 is Na⁺

To a solution of 4,5-dioxo-4,5-dihydro-1H-pyrrolo[2,3-f]quinoline-2,7,9-tricarboxylic acid trimethyl ester (1.0 kg,1 eq, 2.686 moles) in 10% sodium carbonate solution (15 Vol) is heated to about 70 to 75 °C over a period of about 16 hours. Completion of reaction is monitored by HPLC. Thereafter, the reaction mixture is acidified (pH: 3.0-3.5) with 1N hydrochloric acid to precipitate the product [4,5-dioxo-4,5-dihydro-1H-pyrrolo[2,3-f]quinoline-2,7,9-tricarboxylic acid disodium] as dark red solid (about 0.85 Kg, 85%).
**¹H NMR (D₂O, 300 MHz):** 6.84 (s, 1H), 8.48 (s, 1H); **LC**-**MS (ESI):** 329 (M-H), **Purity by HPLC:** 98.6%. **IR (ATR, cm⁻¹) υ:** 3407, 1719, 1666, 1621, 1580, 1537, 1497, 1356, 1243, 975 and 731.

### (B) Preparation of 4,5-dioxo-4,5-dihydro-1H-pyrrolo[2,3-f]quinoline-2,7,9-tricarboxylic acid disodium (PQQ.2Na)

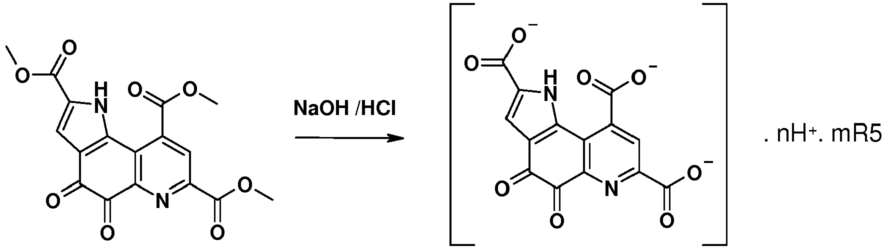

Where in n=1, m=2 and R5 is Na⁺

To a solution of 4,5-dioxo-4,5-dihydro-1H-pyrrolo[2,3-f]quinoline-2,7,9-tricarboxylic acid trimethyl ester (1.0 kg, 1 eq, 2.68 mol) in 3.5% sodium hydroxide solution (15 Vol) is stirred at about 25°C over a period of about 3 hours. Completion of reaction is monitored by HPLC. Thereafter, the reaction mixture is acidified (pH: 3.0-3.5) with 12N hydrochloric acid over a period of about 1h to precipitate the product [4,5-dioxo-4,5-dihydro-1H-pyrrolo[2,3-f]quinoline-2,7,9-tricarboxylic acid disodium] as dark red solid (about 0.88 Kg, 88%).
**¹H NMR** (**D₂O**, **300 MHz):** 6.84 (s, 1H), 8.48 (s, 1H); **LC-MS (ESI):** 329 (M-H), **Purity by HPLC:** 99.4 %. **IR (ATR, cm⁻¹) υ:** 3423, 2558, 1717, 1674, 1611, 1543, 1502, 1235, 1147, 938 and 718.

Based on the above described Examples 1-7, it will be appreciated that the various synthesis steps of intermediate compound (Formula I), PQQ, and the sodium salts of PQQ may be embodied using means for achieving the various solvents and salts. Accordingly, the specification and drawings are to be regarded in an illustrative rather than a restrictive sense.

### Example 8

### Comparative study of the instant process of PQQ synthesis with the prior art

Table 2 provides a comparison showcasing the advantages of the present PQQ synthesis with respect to the known procedures of PQQ synthesis.

**Table 2**

| Prior art reference | Number of steps required to synthesize PQQ | Overall Yield in % | Total time required to get final product in Kg scale |
|---|---|---|---|
| Corey et al.: J. Am. Chem. Soc., 103 (1981), 5599-5600 | 10 | 18-19 | 11-12 weeks |
| Martin et al.: Helv, Chem, Acta 76, (1993), 1667 | 9 | 8-9 | 9-10 weeks |
| WO2006/102642 A1 | 9 | 13-14 | 9-10 weeks |
| Present Invention | 6 | 20-21 | 4-5 weeks |

Based on above table, it can be observed that the instant process of synthesizing PQQ is rapid, significantly reduces cycle time, cost-effective, environmental-friendly and safe when compared to the prior art methods.

In conclusion, the present disclosure provides for compounds of Formula I and tautomers, salts, isomers, analogs, or derivatives thereof, wherein said compounds are immensely useful in various applications such as serving as industrially useful starting materials for the efficient synthesis of several pharmaceutical compounds. In a specific aspect, the present disclosure aims at the syntheses of pyrroloquinoline quinone (PQQ), 5-hydroxy-6-(1,1,4-trioxo-1lambda*6*-1,2,5-thiadiazolidin-2-yl)-1H-indole-2-carboxylic acid, 4,5-dioxo-4,5-dihydro-1H-pyrrolo[2,3-f]quinoline-2,7,9-tricarboxylic acid 2-allyl ester, 5-ethoxy-5-hydroxy-4-oxo-4,5-dihydro-1H-pyrrolo[2,3-f]quinoline-2,7,9-tricarboxylic acid and corresponding salts thereof via Formula I. Further the present disclosure discloses minimal step (6 steps) synthetic process for obtaining the specific compound- Pyrroloquinoline quinone (PQQ) and its salts. The said synthesis routes described in the present disclosure are improved, cost-effective and efficient. Lastly, the specific compound- PQQ synthesized by the present process have numerous applications in neuroprotection, cardioprotection, mitochondrial biogenesis, enhancement of memory, attention and cognition, dietary supplements (nutraceuticals) and so on.

## Claims

1. A compound of Formula I 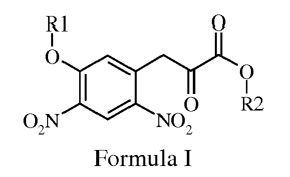 wherein, R1 and R2 are individually selected from a group comprising hydrogen, straight or branched chain C₁₋₈ alkyl, straight or branched chain C₁₋₈ alkenyl, straight or branched chain C₁₋₈ alkynyl, aralkyl, substituted aralkyl, alkaryl, substituted alkaryl, heteroaralkyl, substituted heteroaralkyl and wherein each of them may be optionally substituted,
or tautomers or salts thereof.

2. A process of obtaining a compound of Formula I 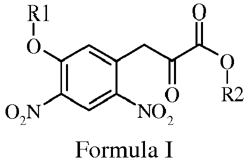 wherein, R1 and R2 are individually selected from a group comprising hydrogen, straight or branched chain C₁₋₈ alkyl, straight or branched chain C₁₋₈ alkenyl, straight or branched chain C₁₋₈ alkynyl, aralkyl, substituted aralkyl, alkaryl, substituted alkaryl, heteroaralkyl, substituted heteroaralkyl and wherein each of them may be optionally substituted, or tautomers or salts thereof, said process comprising reacting a compound of Formula III with ester and base to obtain the compound of Formula I 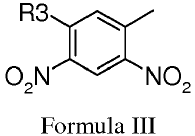 wherein R3 is selected from a group comprising fluoro, chloro, bromo, iodo, methanesulfonyloxy, toluenesulfonyloxy, trifluoromethanesulfonyloxy, alkyloxy, aryloxy, arolkyl oxy, heteroaryloxy and wherein each of them may be optionally substituted.

3. The process as claimed in claim 2, wherein the ester is oxalic acid ester selected from a group comprising dimethyl oxalate, diallyl oxalate, diethyl oxalate, dibenzyl oxalate, diisopropyl oxalate and di-tert-butyl oxalate or any combination thereof; and the base is selected from a group comprising sodium methoxide, sodium ethoxide, sodium benzyloxide, potassium hydroxide, potassium *t*-butoxide, sodium hydride and 1,8-diazabicyclo[5.4.0]undec-7-ene or any combination thereof.

4. The process as claimed in claim 2, wherein the process is carried out at temperature ranging from about -20 °C to about 100 °C, preferably about 0 °C to about 40 °C and for a time period ranging from about 60 minutes to about 72 hours, preferably about 60 minutes to about 16 hours.

5. The process as claimed in claim 2, wherein the process further comprises isolation of the compound of Formula I; and wherein the isolation is carried out by acts selected from a group comprising quenching, filtration and extraction or any combination of acts in any order thereof.

6. A process according to claim 2 for preparing a compound of Formula IV via Formula I, or tautomers or salts thereof, 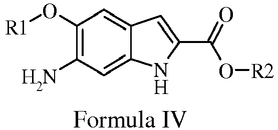 wherein, R1 and R2 are individually selected from a group comprising hydrogen, straight or branched chain C₁₋₈ alkyl, straight or branched chain C₁₋₈ alkenyl, straight or branched chain C₁₋₈ alkynyl, aralkyl, substituted aralkyl, alkaryl, substituted alkaryl, heteroaralkyl, substituted heteroaralkyl and wherein each of them may be optionally substituted,
said process comprising steps of:
a. reacting a compound of Formula II with nitration mixture to obtain a compound of Formula III 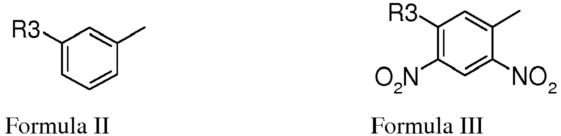 wherein, R3 is selected from a group comprising fluoro, chloro, bromo, iodo, methanesulfonyloxy, toluenesulfonyloxy, trifluoromethanesulfonyloxy, alkyloxy, aryloxy, arolkyl oxy, heteroaryloxy and wherein each of them may be optionally substituted;
b. reacting the compound of Formula III with ester and base to obtain a compound of Formula I, or tautomers or salts thereof 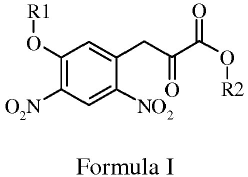 wherein, R1 and R2 are individually selected from a group comprising hydrogen, straight or branched chain C₁₋₈ alkyl, straight or branched chain C₁₋₈ alkenyl, straight or branched chain C₁₋₈ alkynyl, aralkyl, substituted aralkyl, alkaryl, substituted alkaryl, heteroaralkyl, substituted heteroaralkyl and wherein each of them may be optionally substituted; and
c. reacting the compound of Formula I with reducing agent to obtain the compound of Formula IV 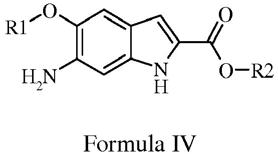 wherein, R1 and R2 are individually selected from a group comprising hydrogen, straight or branched chain C₁₋₈ alkyl, straight or branched chain C₁₋₈ alkenyl, straight or branched chain C₁₋₈ alkynyl, aralkyl, substituted aralkyl, alkaryl, substituted alkaryl, heteroaralkyl, substituted heteroaralkyl and wherein each of them may be optionally substituted.

7. The process as claimed in claim 6, wherein the nitration mixture in step (a) is selected from a group comprising a mixture of nitric acid and sulphuric acid, calcium nitrate and acetic acid, and tert-butyl nitrite or any combination of mixtures thereof; the ester in step (b) is oxalic acid ester selected from a group comprising dimethyl oxalate, diallyl oxalate, diethyl oxalate, Di-*tert*-butyl oxalate, and diisopropyl oxalate or any combination thereof; the base in step (b) is selected from a group comprising sodium methoxide, sodium benzyloxide, sodium ethoxide, potassium hydroxide, potassium *t*-butoxide, sodium hydride and 1,8-diazabicyclo[5.4.0]undec-7-ene or any combination thereof; the reducing agent in step (c) is selected from a group comprising palladium, palladium on carbon, nickel, sodium dithionite, iron/hydrochloric acid, zinc/acetic acid and tin chloride/hydrochloric acid or any combination thereof.

8. The process as claimed in claim 6, wherein the step c) further comprises acts of:
i. converting the compound of Formula IV to a compound of Formula V in presence of coupling agent 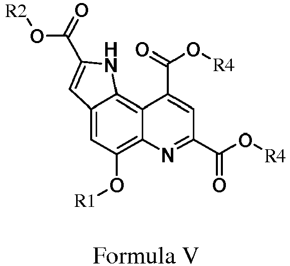 wherein, R1 and R2 are individually selected from a group comprising hydrogen, straight or branched chain C₁₋₈ alkyl, straight or branched chain C₁₋₈ alkenyl, straight or branched chain C₁₋₈ alkynyl, aralkyl, substituted aralkyl, alkaryl, substituted alkaryl, heteroaralkyl, substituted heteroaralkyl and wherein each of them may be optionally substituted; and R4 is selected from group comprising, but not limited to, hydrogen, straight or branched chain C₁₋₈ alkyl, straight or branched chain C₁₋₈ alkenyl, straight or branched chain C₁₋₈ alkynyl, aralkyl, substituted aralkyl, heteroaralkyl, substituted heteroaralkyl and wherein each of them may be optionally substituted;
ii. reacting the compound of Formula V with oxidizing agent to obtain a compound of Formula VI 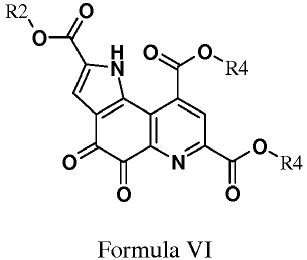 wherein R2 is selected from a group comprising hydrogen, straight or branched chain C₁₋₈ alkyl, straight or branched chain C₁₋₈ alkenyl, straight or branched chain C₁₋₈ alkynyl, aralkyl, substituted aralkyl, alkaryl, substituted alkaryl, heteroaralkyl, substituted heteroaralkyl and wherein each of them may be optionally substituted; and R4 is selected from group comprising hydrogen, straight or branched chain C₁₋₈ alkyl, straight or branched chain C₁₋₈ alkenyl, straight or branched chain C₁₋₈ alkynyl, aralkyl, substituted aralkyl, heteroaralkyl, substituted heteroaralkyl and wherein each of them may be optionally substituted; and
iii. hydrolyzing the compound of Formula VI to obtain 4,5-dioxo-4,5-dihydro-1H-pyrrolo[2,3-f]quinoline-2,7,9-tricarboxylic acid (PQQ) or 4,5-dioxo-4,5-dihydro-1H-pyrrolo[2,3-f]quinoline-2,7,9-tricarboxylic acid 2-allyl ester, or salts thereof.

9. The process as claimed in claim 6, wherein the step c) further comprises acts of:
i. converting the compound of Formula IV to a compound of Formula VII in presence of chlorosulfonyl isocyanate 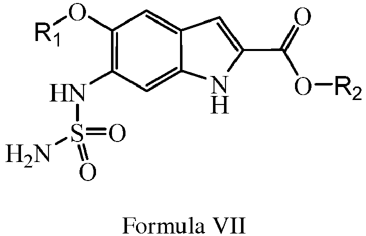 wherein, R1 and R2 are individually selected from a group comprising hydrogen, straight or branched chain C₁₋₈ alkyl, straight or branched chain C₁₋₈ alkenyl, straight or branched chain C₁₋₈ alkynyl, aralkyl, substituted aralkyl, alkaryl, substituted alkaryl, heteroaralkyl, substituted heteroaralkyl and wherein each of them may be optionally substituted; and R4 is selected from group comprising, but not limited to, hydrogen, straight or branched chain C₁₋₈ alkyl, straight or branched chain C₁₋₈ alkenyl, straight or branched chain C₁₋₈ alkynyl, aralkyl, substituted aralkyl, heteroaralkyl, substituted heteroaralkyl and wherein each of them may be optionally substituted; and
ii. reacting the compound of Formula VII with ethyl bromoacetate and palladium on carbon to obtain a compound of 5-hydroxy-6-(1,1,4-trioxo-1lambda*6*-1,2,5-thiadiazolidin-2-yl)-1H-indole-2-carboxylic acid or salts thereof.

10. The process as claimed in claim 6, wherein the step c) further comprises acts of:
i. converting the compound of Formula IV to a compound of Formula V in presence of coupling agent 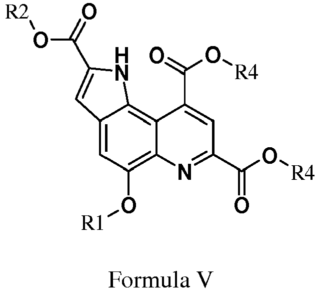 wherein, R1 and R2 are individually selected from a group comprising hydrogen, straight or branched chain C₁₋₈ alkyl, straight or branched chain C₁₋₈ alkenyl, straight or branched chain C₁₋₈ alkynyl, aralkyl, substituted aralkyl, alkaryl, substituted alkaryl, heteroaralkyl, substituted heteroaralkyl and wherein each of them may be optionally substituted; and R4 is selected from group comprising, but not limited to, hydrogen, straight or branched chain C₁₋₈ alkyl, straight or branched chain C₁₋₈ alkenyl, straight or branched chain C₁₋₈ alkynyl, aralkyl, substituted aralkyl, heteroaralkyl, substituted heteroaralkyl and wherein each of them may be optionally substituted;
ii. reacting the compound of Formula V with oxidizing agent to obtain a compound of Formula VI 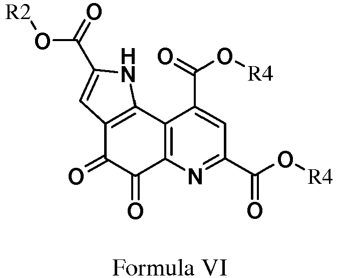 wherein R2 is selected from a group comprising hydrogen, straight or branched chain C₁₋₈ alkyl, straight or branched chain C₁₋₈ alkenyl, straight or branched chain C₁₋₈ alkynyl, aralkyl, substituted aralkyl, alkaryl, substituted alkaryl, heteroaralkyl, substituted heteroaralkyl and wherein each of them may be optionally substituted; and R4 is selected from group comprising hydrogen, straight or branched chain C₁₋₈ alkyl, straight or branched chain C₁₋₈ alkenyl, straight or branched chain C₁₋₈ alkynyl, aralkyl, substituted aralkyl, heteroaralkyl, substituted heteroaralkyl and wherein each of them may be optionally substituted;
iii. hydrolyzing the compound of Formula VI to obtain 4,5-dioxo-4,5-dihydro-1H-pyrrolo[2,3-f]quinoline-2,7,9-tricarboxylic acid (PQQ) or salts thereof; and
iv. refluxing the 4,5-dioxo-4,5-dihydro-1H-pyrrolo[2,3-f]quinoline-2,7,9-tricarboxylic acid (PQQ) in presence of ethanol to obtain 5-ethoxy-5-hydroxy-4-oxo-4,5-dihydro-1H-pyrrolo[2,3-f]quinofine-2,7,9-tricarboxylic acid or salts thereof.

11. The processes as claimed in claim 8, 9 or 10, wherein the salts are selected from a group comprising glucosamine, sodium, calcium and potassium or any combination thereof.

12. The processes as claimed in claim 8 or claim 10 wherein the coupling agent is selected from a group comprising 4-oxo-pent-2-enedioic acid dimethyl ester, copper acetate, 4-oxo-pent-2-enedioic acid diethyl ester, 4-oxo-pent-2-enedioic acid diisopropyl ester and 4-oxo-pent-2-enedioic acid di-tert-butyl ester, or any combination thereof; the oxidizing agent is ceric ammonium nitrate; and the hydrolysis is carried out in presence of base selected from a group comprising sodium carbonate, potassium carbonate, sodium hydroxide and lithium hydroxide, or any combination thereof.

13. The processes as claimed in claim 6, 8, 9 or 10, wherein the process is carried out in presence of solvent selected from a group comprising water, tetrahydrofuran, methanol, benzyl alcohol, ethanol, tertiary butanol, ethyl acetate, acetonitrile, dichloromethane, N,N-dimethylformamide and acetic acid, or any combination thereof, and wherein the process is carried out at temperature ranging from about -20 °C to about 150 °C, preferably about -10 °C to about 100 °C and for a time period ranging from about 30 minutes to about 72 hours, preferably about 30 minutes to about 24 hours.

14. The processes as claimed in claim 8 or claim 10, wherein the step (i) is carried out in presence of acid selected from a group comprising hydrochloric acid, trifluoroacetic acid and p-toluenesulfonic acid, formic acid, or any combination thereof.

15. The processes as claimed in claim 6, 8, 9 or 10, wherein the steps further comprise acts of precipitation or isolation or a combination thereof of the corresponding compound obtained; and wherein the isolation is carried out by acts selected from a group comprising addition of water, quenching, filtration and extraction, or any combination of acts in any order thereof.

## Patentansprüche

1. Verbindung gemäß Formel I, 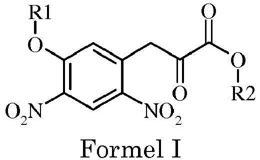 wobei R1 und R2 einzeln ausgewählt sind aus einer Gruppe umfassend Wasserstoff, gerad- oder verzweigtkettiges C₁₋₈ Alkyl, gerad- oder verzweigtkettiges C₁₋₈ Alkenyl, gerad- oder verzweigtkettiges C₁₋₈ Alkinyl, Aralkyl, substituiertes Aralkyl, Alkaryl, substituiertes Alkaryl, Heteroaralkyl, substituiertes Heteroaralkyl und wobei jedes von ihnen optional substituiert sein kann,
oder Tautomere oder Salze davon.

2. Verfahren zum Erhalten einer Verbindung gemäß Formel I, 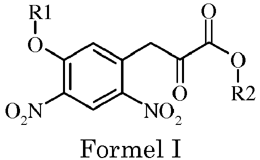 wobei R1 und R2 einzeln ausgewählt sind aus einer Gruppe umfassend Wasserstoff, gerad- oder verzweigtkettiges C₁₋₈ Alkyl, gerad- oder verzweigtkettiges C₁₋₈ Alkenyl, gerad- oder verzweigtkettiges C₁₋₈ Alkinyl, Aralkyl, substituiertes Aralkyl, Alkaryl, substituiertes Alkaryl, Heteroaralkyl, substituiertes Heteroaralkyl und wobei jedes von ihnen optional substituiert sein kann,
oder Tautomere oder Salze davon,
das Verfahren umfassend Reagieren einer Verbindung von Formel III mit Ester und Base, um die Verbindung von Formel I zu erhalten, 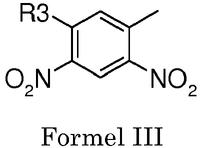 wobei R3 ausgewählt ist aus einer Gruppe umfassend Fluor, Chlor, Brom, Iod, Methansulfonyloxy, Toluensulfonyloxy, Trifluormethansulfonyloxy, Alkyloxy, Aryloxy, Arolkyloxy, Heteroaryloxy und wobei jedes von ihnen optional substituiert sein kann.

3. Verfahren wie in Anspruch 2 beansprucht, wobei der Ester Oxalsäureester ist ausgewählt aus einer Gruppe umfassend Dimethyloxalat, Diallyloxalat, Diethyloxalat, Dibenzyloxalat, Diisopropyloxalat und Di-tert-butyloxalat oder irgendeine Kombination davon; und die Base ausgewählt ist aus einer Gruppe umfassend Natriummethoxid, Natriumethoxid, Natriumbenzyloxid, Kaliumhydroxid, Kalium-*t*-butoxid, Natriumhydrid und 1,8-Diazabicyclo[5.4.0]undec-7-en oder irgendeine Kombination davon.

4. Verfahren wie in Anspruch 2 beansprucht, wobei das Verfahren bei einer Temperatur im Bereich von etwa -20 °C bis etwa 100 °C, bevorzugt etwa 0 °C bis etwa 40 °C und für eine Zeitdauer im Bereich von etwa 60 Minuten bis etwa 72 Stunden, bevorzugt etwa 60 Minuten bis etwa 16 Stunden ausgeführt wird.

5. Verfahren wie in Anspruch 2 beansprucht, wobei das Verfahren ferner Isolieren der Verbindung von Formel I umfasst; und wobei das Isolieren ausgeführt wird durch Handlungen, ausgewählt aus einer Gruppe umfassend Quenchen, Filtration und Extraktion oder irgendeine Kombination von Handlungen in einer beliebigen Reihenfolge davon.

6. Verfahren gemäß Anspruch 2 zur Herstellung einer Verbindung von Formel IV via Formel I, oder Tautomere oder Salze davon, 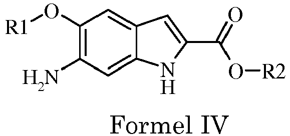 wobei R1 und R2 einzeln ausgewählt sind aus einer Gruppe umfassend Wasserstoff, gerad- oder verzweigtkettiges C₁₋₈ Alkyl, gerad- oder verzweigtkettiges C₁₋₈ Alkenyl, gerad- oder verzweigtkettiges C₁₋₈ Alkinyl, Aralkyl, substituiertes Aralkyl, Alkaryl, substituiertes Alkaryl, Heteroaralkyl, substituiertes Heteroaralkyl und wobei jedes von ihnen optional substituiert sein kann,
das Verfahren umfassend Schritte von:
a. Umsetzen einer Verbindung von Formel II mit einer Nitrierungsmischung um eine Verbindung von Formel III zu erhalten 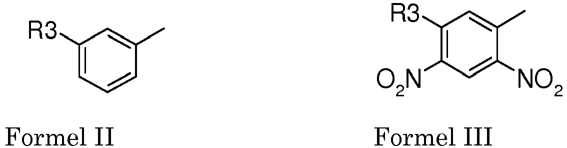 wobei R3 ausgewählt ist aus einer Gruppe umfassend Fluor, Chlor, Brom, Iod, Methansulfonyloxy, Toluensulfonyloxy, Trifluormethansulfonyloxy, Alkyloxy, Aryloxy, Arolkyloxy, Heteroaryloxy und wobei jedes von ihnen optional substituiert sein kann;
b. Umsetzen der Verbindung von Formel III mit Ester und Base um eine Verbindung von Formel I, oder Tautomere oder Salze davon zu erhalten, 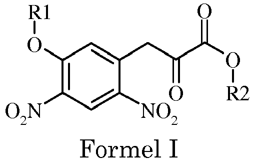 wobei R1 und R2 einzeln ausgewählt sind aus einer Gruppe umfassend Wasserstoff, gerad- oder verzweigtkettiges C₁₋₈ Alkyl, gerad- oder verzweigtkettiges C₁₋₈ Alkenyl, gerad- oder verzweigtkettiges C₁₋₈ Alkinyl, Aralkyl, substituiertes Aralkyl, Alkaryl, substituiertes Alkaryl, Heteroaralkyl, substituiertes Heteroaralkyl und wobei jedes von ihnen optional substituiert sein kann; und
c. Umsetzen der Verbindung von Formel I mit Reduktionsmittel, um die Verbindung von Formel IV zu erhalten, 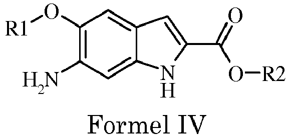 wobei R1 und R2 einzeln ausgewählt sind aus einer Gruppe umfassend Wasserstoff, gerad- oder verzweigtkettiges C₁₋₈ Alkyl, gerad- oder verzweigtkettiges C₁₋₈ Alkenyl, gerad- oder verzweigtkettiges C₁₋₈ Alkinyl, Aralkyl, substituiertes Aralkyl, Alkaryl, substituiertes Alkaryl, Heteroaralkyl, substituiertes Heteroaralkyl und wobei jedes von ihnen optional substituiert sein kann.

7. Verfahren wie in Anspruch 6 beansprucht, wobei die Nitrierungsmischung in Schritt (a) ausgewählt ist aus einer Gruppe umfassend ein Gemisch von Salpetersäure und Schwefelsäure, Calciumnitrat und Essigsäure, und tert-Butylnitrit oder irgendeine Kombination von Gemischen davon; der Ester in Schritt (b) ist Oxalsäureester, ausgewählt aus einer Gruppe umfassend Dimethyloxalat, Diallyloxalat, Diethyloxalat, Di-*tert*-butyloxalat, und Diisopropyloxalat oder irgendeine Kombination davon; die Base in Schritt (b) ist ausgewählt aus einer Gruppe umfassend Natriummethoxid, Natriumbenzyloxid, Natriumethoxid, Kaliumhydroxid, Kalium-*t*-butoxid, Natriumhydrid und 1,8-Diazabicyclo[5.4.0]undec-7-en oder irgendeine Kombination davon; das Reduktionsmittel in Schritt (c) ist ausgewählt aus einer Gruppe umfassend Palladium, Palladium auf Kohlenstoff, Nickel, Natriumdithionit, Eisen/ Salzsäure, Zink/ Essigsäure und Zinnchlorid/ Salzsäure oder irgendeine Kombination davon.

8. Verfahren wie in Anspruch 6 beansprucht, wobei der Schritt c) ferner Handlungen umfasst von:
i. Umsetzen der Verbindung von Formel IV zu einer Verbindung von Formel V in Gegenwart eines Kupplungsmittels 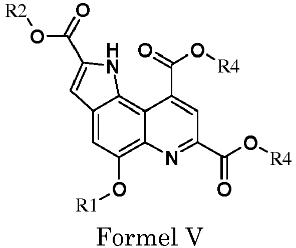 wobei R1 und R2 einzeln ausgewählt sind aus einer Gruppe umfassend Wasserstoff, gerad- oder verzweigtkettiges C₁₋₈ Alkyl, gerad- oder verzweigtkettiges C₁₋₈ Alkenyl, gerad- oder verzweigtkettiges C₁₋₈ Alkinyl, Aralkyl, substituiertes Aralkyl, Alkaryl, substituiertes Alkaryl, Heteroaralkyl, substituiertes Heteroaralkyl und wobei jedes von ihnen optional substituiert sein kann; und R4 ausgewählt ist aus einer Gruppe umfassend, aber nicht beschränkt auf, Wasserstoff, gerad- oder verzweigtkettiges C₁₋₈ Alkyl, gerad- oder verzweigtkettiges C₁₋₈ Alkenyl, gerad- oder verzweigtkettiges C₁₋₈ Alkinyl, Aralkyl, substituiertes Aralkyl, Heteroaralkyl, substituiertes Heteroaralkyl und wobei jedes von ihnen optional substituiert sein kann;
ii. Umsetzen der Verbindung von Formel V mit Oxidationsmittel, um eine Verbindung von Formel VI zu erhalten, 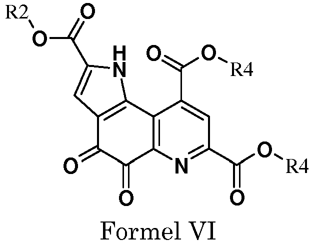 wobei R2 ausgewählt ist aus einer Gruppe umfassend Wasserstoff, gerad- oder verzweigtkettiges C₁₋₈ Alkyl, gerad- oder verzweigtkettiges C₁₋₈ Alkenyl, gerad- oder verzweigtkettiges C₁₋₈ Alkinyl, Aralkyl, substituiertes Aralkyl, Alkaryl, substituiertes Alkaryl, Heteroaralkyl, substituiertes Heteroaralkyl und wobei jedes von ihnen optional substituiert sein kann; und R4 ausgewählt ist aus einer Gruppe umfassend Wasserstoff, gerad- oder verzweigtkettiges C₁₋₈ Alkyl, gerad- oder verzweigtkettiges C₁₋₈ Alkenyl, gerad- oder verzweigtkettiges C₁₋₈ Alkinyl, Aralkyl, substituiertes Aralkyl, Heteroaralkyl, substituiertes Heteroaralkyl und wobei jedes von ihnen optional substituiert sein kann;
und
iii. Hydrolysieren der Verbindung von Formel VI, um 4,5-Dioxo-4,5-dihydro-1H-pyrrolo[2,3-f]chinolin-2,7,9-tricarbonsäure (PQQ) oder 4,5-Dioxo-4,5-dihydro-1H-pyrrolo[2,3-f]chinolin-2,7,9-tricarbonsäure-2-allylester, oder Salze davon zu erhalten.

9. Verfahren wie in Anspruch 6 beansprucht, wobei der Schritt c) ferner Handlungen umfasst von:
i. Umsetzen der Verbindung von Formel IV in eine Verbindung von Formel VII in Gegenwart von Chlorsulfonylisocyanat 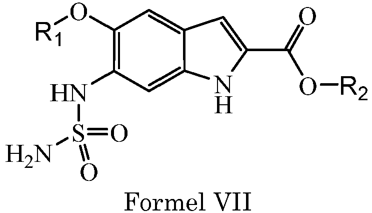 wobei R1 und R2 einzeln ausgewählt sind aus einer Gruppe umfassend Wasserstoff, gerad- oder verzweigtkettiges C₁₋₈ Alkyl, gerad- oder verzweigtkettiges C₁₋₈ Alkenyl, gerad- oder verzweigtkettiges C₁₋₈ Alkinyl, Aralkyl, substituiertes Aralkyl, Alkaryl, substituiertes Alkaryl, Heteroaralkyl, substituiertes Heteroaralkyl und wobei jedes von ihnen optional substituiert sein kann; und R4 ist ausgewählt aus einer Gruppe umfassend, aber nicht beschränkt auf, Wasserstoff, gerad- oder verzweigtkettiges C₁₋₈ Alkyl, gerad- oder verzweigtkettiges C₁₋₈ Alkenyl, gerad- oder verzweigtkettiges C₁₋₈ Alkinyl, Aralkyl, substituiertes Aralkyl, Heteroaralkyl, substituiertes Heteroaralkyl und wobei jedes von ihnen optional substituiert sein kann;
ii. Umsetzen der Verbindung von Formel VII mit Ethylbromacetat und Palladium auf Kohlenstoff, um eine Verbindung von 5-Hydroxy-6-(1,1,4-trioxo-1lambda*6*-1,2,5-thiadiazolidin-2-yl)-1H-indol-2-carbonsäure oder Salze davon zu erhalten.

10. Verfahren wie in Anspruch 6 beansprucht, wobei der Schritt c) ferner Handlungen umfasst von:
i. Umsetzen der Verbindung von Formel IV in eine Verbindung von Formel V in Gegenwart eines Kupplungsmittels, 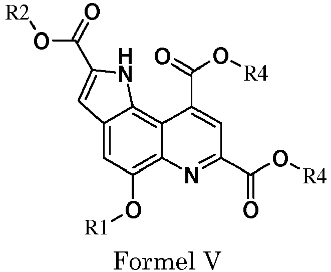 wobei R1 und R2 einzeln ausgewählt sind aus einer Gruppe umfassend Wasserstoff, gerad- oder verzweigtkettiges C₁₋₈ Alkyl, gerad- oder verzweigtkettiges C₁₋₈ Alkenyl, gerad- oder verzweigtkettiges C₁₋₈ Alkinyl, Aralkyl, substituiertes Aralkyl, Alkaryl, substituiertes Alkaryl, Heteroaralkyl, substituiertes Heteroaralkyl und wobei jedes von ihnen optional substituiert sein kann; und R4 ausgewählt ist aus einer Gruppe umfassend, aber nicht beschränkt auf, Wasserstoff, gerad- oder verzweigtkettiges C₁₋₈ Alkyl, gerad- oder verzweigtkettiges C₁₋₈ Alkenyl, gerad- oder verzweigtkettiges C₁₋₈ Alkinyl, Aralkyl, substituiertes Aralkyl, Heteroaralkyl, substituiertes Heteroaralkyl und wobei jedes von ihnen optional substituiert sein kann;
ii. Umsetzen der Verbindung von Formel V mit Oxidationsmittel um eine Verbindung von Formel VI zu erhalten, 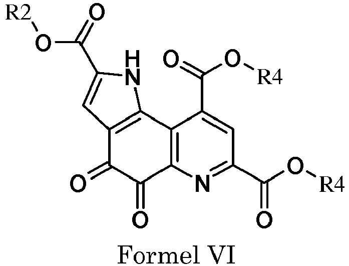 wobei R2 ausgewählt ist aus einer Gruppe umfassend Wasserstoff, gerad- oder verzweigtkettiges C₁₋₈ Alkyl, gerad- oder verzweigtkettiges C₁₋₈ Alkenyl, gerad- oder verzweigtkettiges C₁₋₈ Alkinyl, Aralkyl, substituiertes Aralkyl, Alkaryl, substituiertes Alkaryl, Heteroaralkyl, substituiertes Heteroaralkyl und wobei jedes von ihnen optional substituiert sein kann; und R4 ausgewählt ist aus einer Gruppe umfassend Wasserstoff, gerad- oder verzweigtkettiges C₁₋₈ Alkyl, gerad- oder verzweigtkettiges C₁₋₈ Alkenyl, gerad- oder verzweigtkettiges C₁₋₈ Alkinyl, Aralkyl, substituiertes Aralkyl, Heteroaralkyl, substituiertes Heteroaralkyl und wobei jedes von ihnen optional substituiert sein kann;
iii. Hydrolysieren der Verbindung von Formel VI, um 4,5-Dioxo-4,5-dihydro-1H-pyrrolo[2,3-f]chinolin-2,7,9-tricarbonsäure (PQQ) oder Salze davon zu erhalten; und
iv. Rückflusskochen der 4,5-Dioxo-4,5-dihydro-1H-pyrrolo[2,3-f]chinolin-2,7,9-tricarbonsäure (PQQ) in Gegenwart von Ethanol, um 5-Ethoxy-5-hydroxy-4-oxo-4,5-dihydro-1H-pyrrolo[2,3-f]chinolin-2,7,9-tricarbonsäure oder Salze davon zu erhalten.

11. Verfahren wie in Anspruch 8, 9 oder 10 beansprucht, wobei die Salze ausgewählt sind aus einer Gruppe umfassend Glucosamin, Natrium, Calcium und Kalium oder irgendeine Kombination davon.

12. Verfahren wie in Anspruch 8 oder Anspruch 10 beansprucht, wobei das Kupplungsmittel ausgewählt ist aus einer Gruppe umfassend 4-Oxo-pent-2-endicarbonsäuredimethylester, Kupferacetat, 4-Oxo-pent-2-endicarbonsäurediethylester, 4-Oxo-pent-2-endicarbonsäurediisopropylester und 4-Oxo-pent-2-endicarbonsäuredi-tert-butylester, oder irgendeine Kombination davon; das Oxidationsmittel ist Cerammoniumnitrat; und die Hydrolyse wird durchgeführt in Gegenwart einer Base, ausgewählt aus einer Gruppe umfassend Natriumcarbonat, Kaliumcarbonat, Natriumhydroxid und Lithiumhydroxid, oder irgendeine Kombination davon.

13. Verfahren wie in Anspruch 6, 8, 9 oder 10 beansprucht, wobei das Verfahren in Gegenwart eines Lösungsmittels ausgeführt wird, ausgewählt aus einer Gruppe umfassend Wasser, Tetrahydrofuran, Methanol, Benzylalkohol, Ethanol, tertiäres Butanol, Ethylacetat, Acetonitril, Dichloromethan, N,N-Dimethylformamid und Essigsäure, oder irgendeine Kombination davon, und wobei das Verfahren bei einer Temperatur im Bereich von etwa -20 °C bis etwa 150 °C, bevorzugt etwa -10 °C bis etwa 100 °C und für eine Zeitdauer im Bereich von etwa 30 Minuten bis etwa 72 Stunden, bevorzugt etwa 30 Minuten bis etwa 24 Stunden ausgeführt wird.

14. Verfahren wie in Anspruch 8 oder Anspruch 10 beansprucht, wobei der Schritt (i) in Gegenwart einer Säure ausgeführt wird, ausgewählt aus einer Gruppe umfassend Salzsäure, Trifluoressigsäure und p-Toluensulfonsäure, Ameisensäure, oder irgendeine Kombination davon.

15. Verfahren wie in Anspruch 6, 8, 9 oder 10 beansprucht, wobei die Schritte ferner Handlungen umfassen von Präzipitieren oder Isolieren oder eine Kombination davon von der dazugehörigen erhaltenen Verbindung; und wobei das Isolieren durch Handlungen ausgeführt wird, ausgewählt aus einer Gruppe umfassend Hinzufügen von Wasser, Quenchen, Filtrieren und Extrahieren oder irgendeine Kombination von Handlungen in einer beliebigen Reihenfolge davon.

## Revendications

1. Composé de formule I 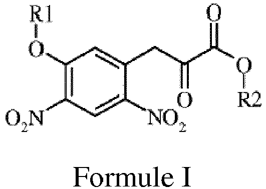 dans laquelle R1 et R2 sont individuellement choisis dans l'ensemble comprenant l'hydrogène, l'alkyle en C₁ à C₈ à chaîne droite ou ramifiée, l'alcényle en C₁ à C₈ à chaîne droite ou ramifiée, l'alcynyle en C₁ à C₈ à chaîne droite ou ramifiée, l'aralkyle, l'aralkyle substitué, l'alcaryle, l'alcaryle substitué, l'hétéroaralkyle, l'hétéroaralkyle substitué, et chacun d'entre eux pouvant être éventuellement substitué,
ou des tautomères ou sels de ce type de composé.

2. Procédé pour obtenir un composé de formule 1 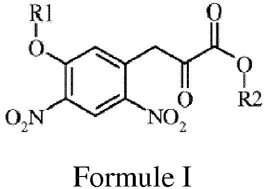 dans laquelle R1 et R2 sont individuellement choisis dans l'ensemble comprenant l'hydrogène, l'alkyle en C₁ à C₈ à chaîne droite ou ramifiée, l'alcényle en C₁ à C₈ à chaîne droite ou ramifiée, l'alcynyle en C₁ à C₈ à chaîne droite ou ramifiée, l'aralkyle, l'aralkyle substitué, l'alcaryle, l'alcaryle substitué, l'hétéroaralkyle, l'hétéroaralkyle substitué, et chacun d'entre eux pouvant être éventuellement substitué,
ou des tautomères ou sels de ce type de composé,
ledit procédé comprenant la réaction d'un composé de formule III avec un ester et une base afin d'obtenir le composé de formule I 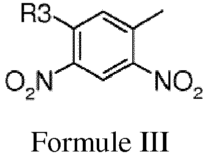 dans laquelle R3 est choisi dans l'ensemble comprenant fluoro, chloro, bromo, iodo, méthanesulfonyloxy, toluènesulfonyloxy, trifluorométhanesulfonyloxy, alkyloxy, aryloxy, aralkyloxy, hétéroaryloxy, et chacun d'entre eux pouvant être éventuellement substitué.

3. Procédé selon la revendication 2, dans lequel l'ester est un ester d'acide oxalique choisi dans l'ensemble comprenant l'oxalate de diméthyle, l'oxalate de diallyle, l'oxalate de diéthyle, l'oxalate de dibenzyle, l'oxalate de diisopropyl et l'oxalate de di-tert-butyle ou l'une quelconque de leurs combinaisons ; et la base est choisie dans l'ensemble comprenant le méthylate de sodium, l'éthylate de sodium, le benzylate de sodium, l'hydroxyde de potassium, le t-butylate de potassium, l'hydrure de sodium et le 1,8-diazabicyclo[5.4.0]undéc-7-ène ou l'une quelconque de leurs combinaisons.

4. Procédé selon la revendication 2, lequel procédé est mis en oeuvre à une température située dans la plage allant d'environ -20°C à environ 100°C, de préférence d'environ 0°C à environ 40°C et sur une période de temps située dans la plage allant d'environ 60 minutes à environ 72 heures, de préférence d'environ 60 minutes à environ 16 heures.

5. Procédé selon la revendication 2, lequel procédé comprend en outre l'isolation du composé de formule I ; et dans lequel l'isolation est mise en oeuvre par des opérations choisies dans l'ensemble comprenant une désactivation, une filtration et une extraction ou n'importe quelle combinaison d'opérations dans n'importe quel ordre.

6. Procédé selon la revendication 2 pour préparer un composé de formule IV via la formule I, ou des tautomères ou sels d'un tel composé, 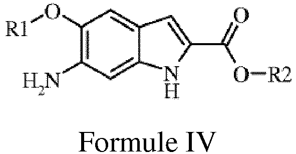 dans laquelle R1 et R2 sont individuellement choisis dans l'ensemble comprenant l'hydrogène, l'alkyle en C₁ à C₈ à chaîne droite ou ramifiée, l'alcényle en C₁ à C₈ à chaîne droite ou ramifiée, l'alcynyle en C₁ à C₈ à chaîne droite ou ramifiée, l'aralkyle, l'aralkyle substitué, l'alcaryle, l'alcaryle substitué, l'hétéroaralkyle, l'hétéroaralkyle substitué, et chacun d'entre eux pouvant être éventuellement substitué,
ledit procédé comprenant les étapes de:
a. faire réagir un composé de formule II avec un mélange de nitration pour obtenir un composé de formule III 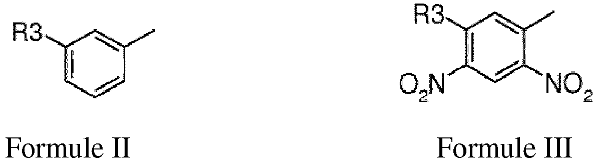 où R3 est choisi dans l'ensemble comprenant le fluoro, chloro, bromo, iodo, méthanesulfonyloxy, le toluènesulfonyloxy, le trifluorométhanesulfonyloxy, l'alkyloxy, l'aryloxy, l'aralkyloxy, l'hétéroaryloxy, et chacun d'entre eux pouvant être éventuellement substitué,
b. faire réagir le composé de formule III avec un ester et une base pour obtenir un composé de formule I, ou des tautomères ou des sels issus de ce composé, 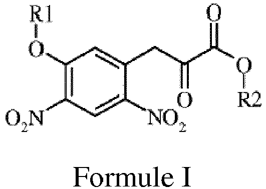 dans laquelle R1 et R2 sont individuellement choisis dans l'ensemble comprenant l'hydrogène, l'alkyle en C₁ à C₈ à chaîne droite ou ramifiée, l'alcényle en C₁ à C₈ à chaîne droite ou ramifiée, l'alcynyle en C₁ à C₈ à chaîne droite ou ramifiée, l'aralkyle, l'aralkyle substitué, l'alcaryle, l'alcaryle substitué, l'hétéroaralkyle, l'hétéroaralkyle substitué, et chacun d'entre eux pouvant être éventuellement substitué ; et
c. faire réagir le composé de formule I avec un agent réducteur pour obtenir le composé de formule IV 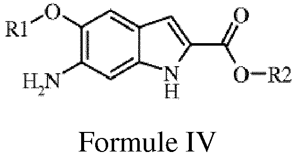 dans laquelle R1 et R2 sont individuellement choisis dans l'ensemble comprenant l'hydrogène, l'alkyle en C₁ à C₈ à chaîne droite ou ramifiée, l'alcényle en C₁ à C₈ à chaîne droite ou ramifiée, l'alcynyle en C₁ à C₈ à chaîne droite ou ramifiée, l'aralkyle, l'aralkyle substitué, l'alcaryle, l'alcaryle substitué, l'hétéroaralkyle, l'hétéroaralkyle substitué, et chacun d'entre eux pouvant être éventuellement substitué.

7. Procédé selon la revendication 6, dans lequel le mélange de nitration de l'étape (a) est choisi dans l'ensemble comprenant un mélange d'acide nitrique et d'acide sulfurique, de nitrate de calcium et d'acide acétique, et de nitrite de tert-butyle ou de n'importe quelle combinaison de mélanges de ceux-ci ; l'ester de l'étape (b) est un ester d'acide oxalique choisi dans l'ensemble comprenant l'oxalate de diméthyle, l'oxalate de diallyle, l'oxalate de diéthyle, l'oxalate de di-tert-butyle, et l'oxalate de diisopropyle ou l'une quelconque de leurs combinaisons; la base de l'étape (b) est choisie dans l'ensemble comprenant le méthylate de sodium, le benzylate de sodium, l'éthylate de sodium, l'hydroxyde de potassium, le t-butylate de potassium, l'hydrure de sodium et le 1,8-diazabicyclo[5.4.0]undéc-7-ène ou l'une quelconque de leurs combinaisons; l'agent réducteur de l'étape (c) est choisi dans l'ensemble comprenant le palladium, le charbon palladié, le nickel, le dithionite de sodium, le fer/acide chlorhydrique, le zinc/acide acétique et le chlorure d'étain/acide chlorhydrique ou l'une quelconque de leurs combinaisons.

8. Procédé selon la revendication 6, dans lequel l'étape c) comprend en outre les opérations de:
i. convertir le composé de formule IV en un composé de formule V en présence d'un agent de couplage, 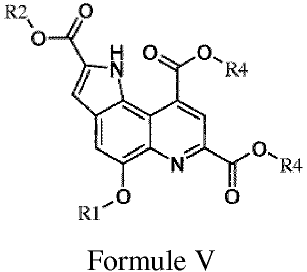 dans laquelle R1 et R2 sont individuellement choisis dans l'ensemble comprenant l'hydrogène, l'alkyle en C₁ à C₈ à chaîne droite ou ramifiée, l'alcényle en C₁ à C₈ à chaîne droite ou ramifiée, l'alcynyle en C₁ à C₈ à chaîne droite ou ramifiée, l'aralkyle, l'aralkyle substitué, l'alcaryle, l'alcaryle substitué, l'hétéroaralkyle, l'hétéroaralkyle substitué, et chacun d'entre eux pouvant être éventuellement substitué; et R4 est choisi dans l'ensemble comprenant, mais sans s'y limiter, l'hydrogène, alkyle en C₁ à C₈ à chaîne droite ou ramifiée, alcényle en C₁ à C₈ à chaîne droite ou ramifiée, alcynyle en C₁ à C₈ à chaîne droite ou ramifiée, aralkyle, aralkyle substitué, hétéroaralkyle, hétéroaralkyle substitué, chacun d'entre eux pouvant être éventuellement substitué ;
ii. faire réagir le composé de formule V avec un agent oxydant pour obtenir un composé de formule VI, 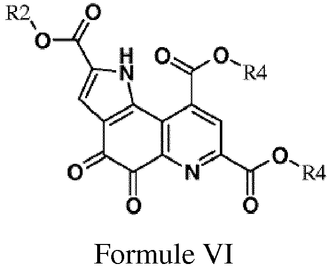 dans laquelle R2 est choisi dans l'ensemble comprenant l'hydrogène, l'alkyle en C₁ à C₈ à chaîne droite ou ramifiée, l'alcényle en C₁ à C₈ à chaîne droite ou ramifiée, l'alcynyle en C₁ à C₈ à chaîne droite ou ramifiée, l'aralkyle, l'aralkyle substitué, l'alcaryle, l'alcaryle substitué, l'hétéroaralkyle, l'hétéroaralkyle substitué, et chacun d'entre eux pouvant être éventuellement substitué ; et R4 est choisi dans l'ensemble comprenant l'hydrogène, alkyle en C₁ à C₈ à chaîne droite ou ramifiée, alcényle en C₁ à C₈ linéaire ou ramifié, alcynyle en C₁ à C₈ linéaire ou ramifié, aralkyle, aralkyle substitué, hétéroaralkyle, hétéroaralkyle substitué, chacun d'entre eux pouvant être éventuellement substitué;
et
iii. hydrolyser le composé de formule VI pour obtenir de l'acide 4,5-dioxo-4,5-dihydro-1H-pyrrolo[2,3-f]quinoline-2,7,9-tricarboxylique (PQQ) ou de l'ester 2-allylique d'acide 4,5-dioxo-4,5-dihydro-1H-pyrrolo[2,3-f]quinoline-2,7,9-tricarboxylique, ou des sels de ceux-ci.

9. Procédé selon la revendication 6, dans lequel l'étape c) comprend en outre les opérations de:
i. convertir le composé de formule IV en un composé de formule VII en présence d'isocyanate de chlorosulfonyle, 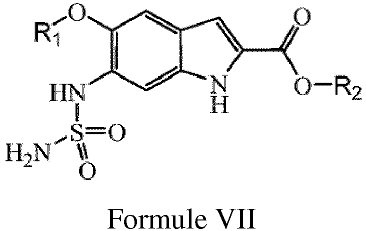 dans laquelle R1 et R2 sont individuellement choisis dans l'ensemble comprenant l'hydrogène, l'alkyle en C₁ à C₈ à chaîne droite ou ramifiée, l' alcényle en C₁ à C₈ à chaîne droite ou ramifiée, l'alcynyle en C₁ à C₈ à chaîne droite ou ramifiée, l'aralkyle, l'aralkyle substitué, l'alcaryle, l'alcaryle substitué, l'hétéroaralkyle, l'hétéroaralkyle substitué, et chacun d'entre eux pouvant être éventuellement substitué, et R4 est choisi dans l'ensemble comprenant, mais sans s'y limiter, l'hydrogène, l'alkyle en C₁ à C₈ à chaîne droite ou ramifiée, l'alcényle en C₁ à C₈ à chaîne droite ou ramifiée, l'alcynyle en C₁ à C₈ à chaîne droite ou ramifiée, l'aralkyle, l'aralkyle substitué, l'hétéroaralkyle, l'hétéroaralkyle substitué, et chacun d'entre eux pouvant être éventuellement substitué ; et
ii. faire réagir le composé de formule VII avec du bromoacétate d'éthyle et du charbon palladié pour obtenir un composé d'acide 5-hydroxy-6-(1,1,4-trioxo-1lambda*6*-1,2,5-thiadiazolidin-2-yl)-1H-indole-2-carboxylique ou des sels de celui-ci.

10. Procédé selon la revendication 6, dans lequel l'étape c) comprend en outre les opérations de:
i. convertir le composé de formule IV en un composé de formule V en présence d'un agent de couplage, 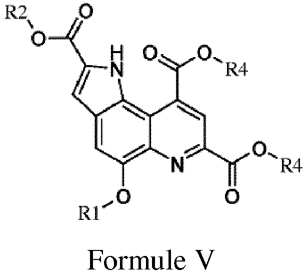 dans laquelle R1 et R2 sont individuellement choisis dans l'ensemble comprenant l'hydrogène, l'alkyle en C₁ à C₈ à chaîne droite ou ramifiée, l'alcényle en C₁ à C₈ à chaîne droite ou ramifiée, l'alcynyle en C₁ à C₈ à chaîne droite ou ramifiée, l'aralkyle, l'aralkyle substitué, l'alcaryle, l'alcaryle substitué, l'hétéroaralkyle, l'hétéroaralkyle substitué, et chacun d'entre eux pouvant être éventuellement substitué; et R4 est choisi dans l'ensemble comprenant, mais sans s'y limiter, l'hydrogène, l'alkyle en C₁ à C₈ à chaîne droite ou ramifiée, l'alcényle en C₁ à C₈ à chaîne droite ou ramifiée, l'alcynyle en C₁ à C₈ à chaîne droite ou ramifiée, l'aralkyle, l'aralkyle substitué, l'hétéroaralkyle, l'hétéroaralkyle substitué, chacun d'entre eux pouvant être éventuellement substitué;
ii. faire réagir le composé de formule V avec un agent oxydant pour obtenir un composé de formule VI, 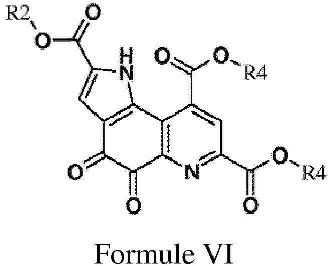 dans laquelle R2 est individuellement choisi dans l'ensemble comprenant l'hydrogène, l'alkyle en C₁ à C₈ à chaîne droite ou ramifiée, l'alcényle en C₁ à C₈ à chaîne droite ou ramifiée, l'alcynyle en C₁ à C₈ à chaîne droite ou ramifiée, l'aralkyle, l'aralkyle substitué, l'alcaryle, l'alcaryle substitué, l'hétéroaralkyle, l'hétéroaralkyle substitué, et chacun d'entre eux pouvant être éventuellement substitué ; et R4 est choisi dans l'ensemble comprenant l'hydrogène, l'alkyle en C₁ à C₈ à chaîne droite ou ramifiée, l'alcényle en C₁ à C₈ à chaîne droite ou ramifiée, l'alcynyle en C₁ à C₈ à chaîne droite ou ramifiée, l'aralkyle, l'aralkyle substitué, l'hétéroaralkyle, l'hétéroaralkyle substitué, et chacun d'entre eux pouvant être éventuellement substitué;
iii. hydrolyser le composé de formule VI pour obtenir de l'acide 4,5-dioxo-4,5-dihydro-1H-pyrrolo[2,3-f]quinoline-2,7,9-tricarboxylique (PQQ) ou des sels de celui-ci; et
iv. porter au reflux l'acide 4,5-dioxo-4,5-dihydro-1H-pyrrolo[2,3-f]quinoline-2,7,9-tricarboxylique (PQQ) en présence d'éthanol pour obtenir de l'acide 5-éthoxy-5-hydroxy-4-oxo-4,5-dihydro-1H-pyrrolo[2,3-f]quinoline-2,7,9-tricarboxylique ou des sels de celui-ci.

11. Procédé selon la revendication 8, 9 ou 10, dans lequel les sels sont choisis dans l'ensemble constitué par la glucosamine, le sodium, le calcium et le potassium, ou l'une quelconque de leurs combinaisons.

12. Procédé selon la revendication 8 ou 10, dans lequel l'agent de couplage est choisi dans l'ensemble comprenant l'ester diméthylique d'acide 4-oxopent-2-ènedioïque, l'acétate de cuivre, l'ester diéthylique d'acide 4-oxopent-2-ènedioïque, l'ester dipropylique d'acide 4-oxopent-2-ènedioïque et l'ester di-tert-butylique d'acide 4-oxopent-2-ènedioïque, ou l'une quelconque de leurs combinaisons; l'agent oxydant est le nitrate d'ammonium cérique; et l'hydrolyse est mise en oeuvre en présence d'une base choisie dans l'ensemble comprenant le carbonate de sodium, le carbonate de potassium, l'hydroxyde de sodium et l'hydroxyde de lithium, ou l'une quelconque de leurs combinaisons.

13. Procédé selon la revendication 6, 8, 9 ou 10, lequel procédé est mis en oeuvre en présence d'un solvant choisi dans l'ensemble comprenant l'eau, le tétrahydrofurane, le méthanol, l'alcool benzylique, l'éthanol, le tert-butanol, l'acétate d'éthyle, l'acétonitrile, le dichlorométhane, le N,N-diméthylformamide et l'acide acétique, ou l'une quelconque de leurs combinaisons, et lequel procédé est mis en oeuvre à une température située dans la plage allant d'environ -20°C à environ 150°C, de préférence d'environ -10°C à environ 100°C et pendant une période de temps située dans la plage allant d'environ 30 minutes à environ 72 heures, de préférence d'environ 30 minutes à environ 24 heures.

14. Procédé selon la revendication 8 ou la revendication 10, dans lequel l'étape (i) est mise en oeuvre en présence d'un acide choisi dans l'ensemble comprenant l'acide chlorhydrique, l'acide trifluoroacétique et l'acide p-toluènesulfonique, l'acide formique, ou l'une quelconque de leurs combinaisons.

15. Procédé selon la revendication 6, 8, 9 ou 10, dans lequel les étapes comprennent en outre les opérations de précipitation ou d'isolation ou d'une de leurs combinaisons du composé correspondant obtenu; et dans lequel l'isolation est mise en oeuvre par des opérations choisies dans l'ensemble comprenant une addition d'eau, une désactivation, une filtration et une extraction, ou n'importe quelle combinaison d'opérations quelle qu'en soit l'ordre.
